# EUROPEAN PATENT APPLICATION

(11) **EP 4 585 689 A1**
(43) Date of publication of application: **16.07.2025**
(21) Application number: 23863474.5
(22) Date of filing: 05.09.2023
(51) Int. Cl.: C12N 15/63, C12N 15/113, C12N 9/22

(54) **NOVEL GENOMIC SAFE HARBOR AND USE THEREOF**

(30) Priority: 05.09.2022 KR 20220112185
(71) Applicant: Daewoong Pharmaceutical Co., Ltd., Hwaseong-si, Gyenoggi-do 18623 (KR); AffyXell Therapeutics Co., Ltd., Gimhae-si, Gyeongsangnam-do 50969 (KR)
(72) Inventor: CHOI, Yong Jin, Suwon-si Gyeonggi-do 16707 (KR); KIM, Ki Cheol, Suwon-si Gyeonggi-do 16509 (KR); AHN, Kyong Hoon, Gimhae-si Gyeongsangnam-do 50969 (KR); RYU, Jong Sang, Gimhae-si Gyeongsangnam-do 50969 (KR)
(74) Representative: Mewburn Ellis LLP
(86) International application number: PCT/KR2023/013272
(87) International publication number: WO 2024/054006

(57) **Abstract**

The present invention relates to a novel genomic safe harbor (GSH), a method for expressing a transgene using same, and cells introduced with a transgene by the same method. The method for expressing a transgene using the novel genomic safe harbor according to the present invention allows the safe introduction and long-term stable expression of a transgene without disrupting the transcription of adjacent genes, thereby enabling the safe expression of various transgenes within cells without safety concerns.

## Description

### [Technical Field]

The present invention relates to a novel genomic safe harbor (GSH) for gene insertion and a method of expressing transgenes using the same. Specifically, the present invention relates to a novel genomic safe harbor (GSH) for gene insertion that is capable of expressing various transgenes in cells without safety issues by safely introducing the transgenes and stably expressing the transgenes, and a method of expressing transgenes using the same.

### [Background Art]

Insertion of transgenes into the human genome is a powerful means for treating human diseases. Genes inserted into human cells may change the characteristics of host cells and thus may be applied to treatment of diseases. However, technologies for accurate delivery and continuous expression of the genes should be applied.

The most common method for inserting genes into the human genome is based on the use of retroviral or lentiviral vectors. However, these vectors tend to be inserted around actively transcribed genes, thus increasing the possibility of causing mutations in the host cells. When mutations occur in cancer-related genes and expression is disrupted, various types of cancer may develop.

As an alternative to viral vectors, a site-specific gene insertion system based on CRISPR-Cas9 and homology-directed repair (HDR) is used. CRISPR stands for clustered regularly interspaced short palindromic repeats and is a genetic sequence that acts as an adaptive immune system in bacteria. When bacteria are infected with a virus, the guide RNA (gRNA) expressed from CRISPR binds to the nuclease Cas9, and recognizes and cleaves the part of the virus genome that has a sequence complementary to the gRNA, to prevent infection. HDR is a process of repairing damaged DNA using DNA fragments that are homologous to the corresponding position sequence when a double strand break (DSB) occurs. By inducing DSB in the genome through gRNA and Cas9, and simultaneously introducing a donor vector that has a sequence homologous to transgenes, gene insertion can be precisely induced.

As technologies such as CRISPR-Cas9 are used, exploration of regions of the genome suitable for introduction of transgenes is also underway. A region where the expression of transgenes can be maintained without disrupting the transcription of adjacent genes is called a "genomic safe harbor" (GSH). It is known that the previously studied adeno-associated virus integration site 1 (AAVS1) on chromosome 19 is used as the GSH, but it is located in a region where genes are densely packed and thus may disrupt transcription of adjacent genes (Sadelain et al., 2012), and that the expression of introduced transgenes tends to be suppressed (Ordovas et al., 2015). Therefore, it is necessary to discover novel GSH candidates for safe introduction and stable expression of transgenes.

The present inventors conducted various studies to find a region suitable for a novel genomic safe harbor. As a result, the present inventors found a novel genomic safe harbor region that does not disrupt transcription of adjacent genes and stably expresses transgenes. Based on this finding, the present invention has been completed.

### [Disclosure]

### [Technical Problem]

Therefore, the present invention has been made in view of the above problems, and it is one object of the present invention to provide a novel genomic safe harbor for safely introducing and stably expressing transgenes.

It is another object of the present invention to provide a method of expressing transgenes in cells using the novel genomic safe harbor.

### [Technical Solution]

In accordance with an aspect of the present invention, the above and other objects can be accomplished by the provision of a method of expressing a exogenous polynucleotide in a cell, the method comprising (1) expressing a nuclease by introducing a polynucleotide encoding the nuclease into the cell, (2) cleaving a nucleic acid region by specifically binding the nuclease to at least one nucleic acid region selected from the group consisting of a first nucleic acid region at positions 24894446 to 24894525 in chromosome 9 of the human genome, a second nucleic acid region at positions 9064276 to 9064355 of chromosome 3, and a third nucleic acid region at positions 120174229 to 120174308 in chromosome 4, and (3) introducing a exogenous polynucleotide into the cell and inserting the exogenous polynucleotide into a cleavage site of the nucleic acid region.

In an embodiment, the cell may be derived from human blood, bodily fluid, tissue, stem cells, or cancer.

In an embodiment, the cell may be a somatic cell, a germ cell, a stem cell, a cancer cell, or a cell line.

In an embodiment, the nuclease may comprise at least one selected from zinc finger nucleases, transcription activator-like effector nucleases (TALENs), and RNA-guided engineered nucleases (RGENs).

In an embodiment, the nuclease may be Cas9.

In an embodiment, the polynucleotide encoding the nuclease may comprise at least one selected from a DNA binding domain, a guide RNA, and a cleavage domain.

In an embodiment, the method may further comprise introducing a polynucleotide encoding a guide RNA into the cell.

In an embodiment, the exogenous polynucleotide may comprise a polynucleotide encoding a polypeptide or a polynucleotide encoding a functional polyribonucleotide.

In an embodiment, the functional polyribonucleotide may comprise at least one selected from the group consisting of micro RNA (miRNA), short hairpin RNA (shRNA), piRNA, small nucleolar RNA (snoRNA), small nuclear RNA (snRNA), and extracellular RNA (exRNA) .

In an embodiment, the exogenous polynucleotide may encode at least one selected from the group consisting of antibodies, enzymes, growth factors, receptors, hormones, lymphokines, cytokines, signaling factors, reporters, and fragments thereof.

In an embodiment, the exogenous polynucleotide may be a sonic hedgehog (SHH) gene.

In an embodiment, the exogenous polynucleotide may comprise at least one selected from the group consisting of an open reading frame, a polyadenylation sequence, a promoter, an operator, an enhancer, a transcriptional regulatory element, a signal sequence, and at least one homology region.

In an embodiment, the method may further comprise introducing a left homology arm (LHA), which is a region binding to a region to up to 1 kb away from the left of at least one of cleavage sites of the first to third nucleic acid regions, and a right homology arm (RHA), which is a region binding to a region to up to 1 kb away from the right of at least one of the cleavage sites of the first to third nucleic acid regions, into the exogenous polynucleotide after (2) cleaving a nucleic acid region by specifically binding the nuclease to at least one nucleic acid region selected from the group consisting of a first nucleic acid region at positions 24894446 to 24894525 in chromosome 9 of the human genome, a second nucleic acid region at positions 9064276 to 9064355 of chromosome 3, and a third nucleic acid region at positions 120174229 to 120174308 in chromosome 4.

In an embodiment, the method may further comprise introducing a left homology arm (LHA), which is a region binding to a region to up to 0.8 kb away from the left of at least one of cleavage sites of the first to third nucleic acid regions, and a right homology arm (RHA), which is a region binding to a region to up to 0.8 kb away from the right of at least one of the cleavage sites of the first to third nucleic acid regions, into the exogenous polynucleotide after (2) cleaving a nucleic acid region by specifically binding the nuclease to at least one nucleic acid region selected from the group consisting of a first nucleic acid region at positions 24894446 to 24894525 in chromosome 9 of the human genome, a second nucleic acid region at positions 9064276 to 9064355 of chromosome 3, and a third nucleic acid region at positions 120174229 to 120174308 in chromosome 4.

In accordance with another aspect of the present invention, there is provided a cell having a exogenous polynucleotide inserted into a genome of a cell by at least one nuclease, wherein the exogenous polynucleotide is inserted into at least one selected from a first nucleic acid region from positions 24894446 to 24894525 in chromosome 9 of the human genome, a second nucleic acid region from positions 9064276 to the 9064355 in chromosome 3, and a third nucleic acid region from positions 120174229 to 120174308 in chromosome 4 in the genome of the cell.

In accordance with another aspect of the present invention, there is provided a composition for expressing a exogenous polynucleotide in a cell, the composition containing a polynucleotide encoding a nuclease that specifically binds to at least one selected from a first nucleic acid region from positions 24894446 to 24894525 in chromosome 9 of the human genome, a second nucleic acid region from positions 9064276 to 9064355 in chromosome 3, and a third nucleic acid region from positions 120174229 to 120174308 in chromosome 4 and a exogenous polynucleotide.

In an embodiment, the composition may further contain a left homology arm (LHA), which is a region binding to a region to up to 1 kb away from the left of at least one of cleavage sites of the first to third nucleic acid regions, and a right homology arm (RHA), which is a region binding to a region to up to 1 kb away from the right of at least one of the cleavage sites of the first to third nucleic acid regions.

In an embodiment, the exogenous polynucleotide may be a sonic hedgehog (SHH) gene.

In accordance with a further aspect of the present invention, there is provided a polynucleotide comprising a sequence having a sequence homology of at least 90%, at least 95% or at least 99% with any one of sequences of SEQ ID NOs: 3 to 5.

The present invention, there is also provided a polynucleotide may comprise any one of sequences of SEQ ID NOs: 3 to 5.

In an embodiment, the sequence of SEQ ID NO: 3 may specifically bind to a nucleic acid region at positions 24894446 to 24894525 in chromosome 9 of the human genome.

In an embodiment, the sequence of SEQ ID NO: 4 may specifically bind to a nucleic acid region at positions 9064276 to 9064355 in chromosome 3 of the human genome.

In an embodiment, the sequence of SEQ ID NO: 5 may specifically bind to a nucleic acid region at positions 120174229 to 120174308 in chromosome 4 of the human genome.

In accordance with a further aspect of the present invention, there is provided a polynucleotide comprising sequences of SEQ ID NOs: 14 and 15.

In accordance with a further aspect of the present invention, there is provided a polynucleotide comprising sequences of SEQ ID NOs: 16 and 17.

In accordance with a further aspect of the present invention, there is provided a polynucleotide comprising sequences of SEQ ID NOs: 17 and 18.

In accordance with a further aspect of the present invention, there is provided a vector comprising the polynucleotide described above.

### [Advantageous effects]

The genomic safe harbor of the present invention is capable of stably maintaining the expression of transgenes without disturbing the transcription of adjacent genes even when the transgenes are introduced. Therefore, the genomic safe harbor of the present invention may be used for intracellular expression of various transgenes.

### [Description of Drawings]

FIG. 1 is a schematic diagram illustrating a gRNA/Cas9 expression vector of the present invention.
FIG. 2 is a schematic diagram illustrating a donor vector of the present invention.
FIG. 3 is a schematic diagram illustrating a process of producing a gRNA/Cas9 expression vector targeting AAVS1 and GSH1 to GSH3 of the present invention.
FIG. 4 is a schematic diagram illustrating a process of producing a donor vector of the present invention.
FIG. 5 is a schematic diagram illustrating a process of introducing transgenes using the gRNA/Cas9 expression vector of the present invention and the donor vector.
FIG. 6 is a graph showing mean fluorescence intensity of cells into which GFP genes have been introduced.
FIG. 7 is a graph showing expression changes of AAVS and GSH1 to GSH3 adjacent genes of the present invention.
FIG. 8 is a schematic diagram illustrating a process of introducing SHH genes into the GSH3 of ES-MSC.
FIG. 9 is a graph showing SHH mRNA expression of SHH-overexpressing ES-MSC (SHH-ES-MSC) and an image showing hair follicle regeneration ability of SHH-overexpressing ES-MSC (SHH-ES-MSC) .

### [Best Mode]

Hereinafter, with reference to the attached drawings, embodiments and examples of the present invention will be described in detail so that those skilled in the art can easily implement the present invention. However, the present invention may be implemented in various forms and is not limited to the embodiments and examples described herein.

It will be further understood that, as used herein, the term "comprise" specifies the presence of another element, but do not preclude the presence or addition of other element, unless defined otherwise.

As used herein, the term "genomic safe harbor" refers to a region that can maintain the expression of transgenes without disrupting the transcription of adjacent genes.

As used herein, the term "nuclease" refers to a protein used for genome editing or gene editing and examples thereof comprise, but are not limited to, zinc finger nuclease, TALENs (transcription activator-like effector nuclease), and RGENs (RNA-guided engineered nucleases).

As used herein, the term "exogenous polynucleotide" refers to a polynucleotide that is artificially introduced.

As used herein, the term "guide RNA" refers to RNA specific to target DNA, complementarily binds to the target DNA and allows a nuclease to cleave the target DNA.

As used herein, the term "left homology arm (LHA)" means a sequence that is capable of binding to the left end of a site cleaved by a nuclease in human genomic DNA, and is preferably 1,000 base pairs, more preferably 800 base pairs.

As used herein, the term "right homology arm (RHA)" means a sequence that is capable of binding to the right end of a site cleaved by a nuclease in human genomic DNA, and is preferably 1,000 base pairs, more preferably 800 base pairs.

As used herein, the term "vector" refers to a construct capable of delivering, and preferably expressing, one or more gene(s) or sequence(s) of interest into host cells. Examples of the vector comprise, but are not limited to, viral vectors, naked DNA or RNA expression vectors, plasmids, cosmids, or phage vectors, DNA or RNA expression vectors associated with cationic condensing agents, DNA or RNA expression vectors encapsulated in liposomes, and certain eukaryotic cells, such as producer cells.

As used herein, the term "prevention" means any action that inhibits or delays the onset of a disease by administration of a composition and the term "treatment" means any action that ameliorates or beneficially alters symptoms of a subject suspected of having a disease and having an onset of a disease by administration of a composition.

Hereinafter, the present invention will be described in more detail with reference to examples. However, it will be obvious to those skilled in the art that these examples are provided only for illustration of the present invention and should not be construed as limiting the scope of the present invention.

### [Example 1]

### Production of vector for expression of genomic safe harbor

A gRNA/Cas9 expression vector of SEQ ID NO. 1 which contains a gRNA sequence and thus can express Cas9 protein, and induces DNA double-strand break (DSB) because Cas9 acts on a sequence complementary to gRNA was prepared.

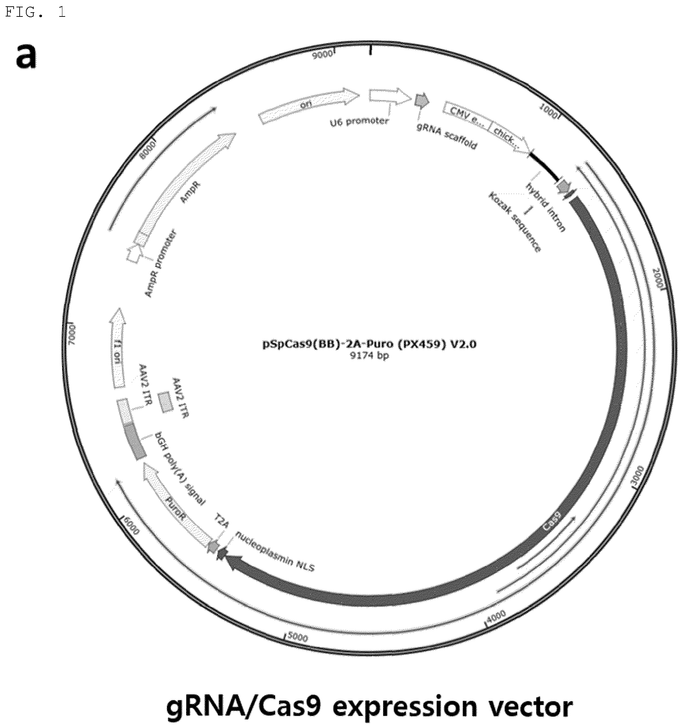

As shown in FIG. 1, the gRNA/Cas9 expression vector contains an origin of replication (ori), U6 promoter, gRNA scaffold, cytomegalovirus promoter (CMV promoter), chicken beta actin promoter, Cas9 gene, puromycin resistance gene, Poly(A) tail, and ampicillin resistance gene.

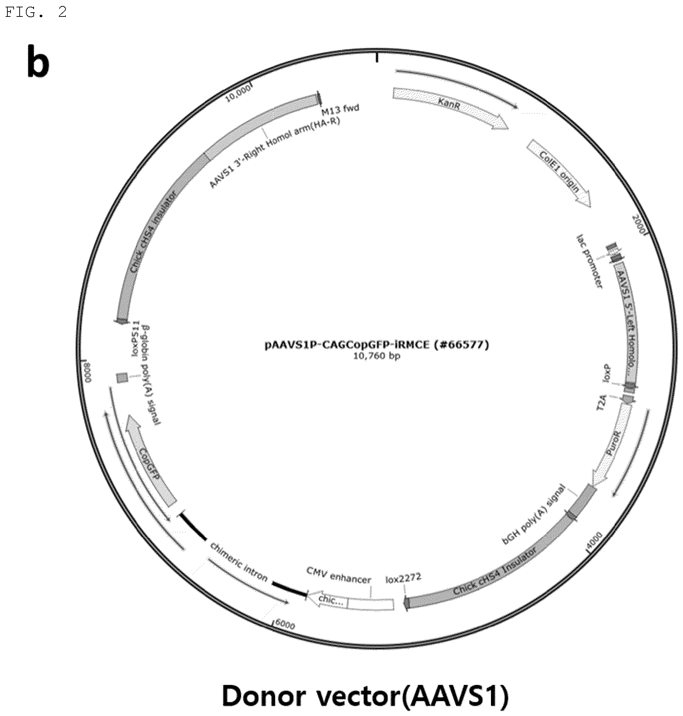

A donor vector of SEQ ID NO. 2 to insert the gene sequence encoding the target protein into GSH was prepared. In this study, the efficiency of the system was measured by inserting a gene sequence encoding a green fluorescent protein (GFP) protein that fluoresces. As shown in FIG. 2, the donor vector comprises an origin of replication, a GSH homology region, a puromycin resistance gene, a chicken beta actin promoter, a cytomegalovirus promoter, a GFP gene, a cHS4 insulator, and a kanamycin resistance gene, wherein the homology region was designed to vary depending on the position of the GSH.

**[Table 1]**

| SEQ ID NO: | Name | Base sequence |
|---|---|---|
| 1 | gRNA/Cas9 expression | GAGGGCCTATTTCCCATGATTCCTTCATATTTGCATATAC GATACAAGGCTGTTAGAGAGATAATTGGAATTAATTTGA |
| | vector | CTGTAAACACAAAGATATTAGTACAAAATACGTGACGTA GAAAGTAATAATTTCTTGGGTAGTTTGCAGTTTTAAAATT ATGTTTTAAAATGGACTATCATATGCTTACCGTAACTTGA AAGTATTTCGATTTCTTGGCTTTATATATCTTGTGGAAAG GACGAAACACCGGGTCTTCGAGAAGACCTGTTTTAGAGC TAGAAATAGCAAGTTAAAATAAGGCTAGTCCGTTATCAA CTTGAAAAAGTGGCACCGAGTCGGTGCTTTTTTGTTTTAG AGCTAGAAATAGCAAGTTAAAATAAGGCTAGTCCGTTTT TAGCGCGTGCGCCAATTCTGCAGACAAATGGCTCTAGAG GTACCCGTTACATAACTTACGGTAAATGGCCCGCCTGGCT GACCGCCCAACGACCCCCGCCCATTGACGTCAATAGTAA CGCCAATAGGGACTTTCCATTGACGTCAATGGGTGGAGT ATTTACGGTAAACTGCCCACTTGGCAGTACATCAAGTGTA TCATATGCCAAGTACGCCCCCTATTGACGTCAATGACGGT AAATGGCCCGCCTGGCATTGTGCCCAGTACATGACCTTAT GGGACTTTCCTACTTGGCAGTACATCTACGTATTAGTCAT CGCTATTACCATGGTCGAGGTGAGCCCCACGTTCTGCTTC ACTCTCCCCATCTCCCCCCCCTCCCCACCCCCAATTTTGTA TTTATTTATTTTTTAATTATTTTGTGCAGCGATGGGGGCGGG GGGGGGGGGGGGGCGCGCGCCAGGCGGGGCGGGGCGG GGCGAGGGGCGGGGCGGGGCGAGGCGGAGAGGTGCGGC GGCAGCCAATCAGAGCGGCGCGCTCCGAAAGTTTCCTTTT ATGGCGAGGCGGCGGCGGCGGCGGCCCTATAAAAAGCG AAGCGCGCGGCGGGCGGGAGTCGCTGCGCGCTGCCTTCG CCCCGTGCCCCGCTCCGCCGCCGCCTCGCGCCGCCCGCCC CGGCTCTGACTGACCGCGTTACTCCCACAGGTGAGCGGG CGGGACGGCCCTTCTCCTCCGGGCTGTAATTAGCTGAGCA AGAGGTAAGGGTTTAAGGGATGGTTGGTTGGTGGGGTAT TAATGTTTAATTACCTGGAGCACCTGCCTGAAATCACTTT TTTTCAGGTTGGACCGGTGCCACCATGGACTATAAGGACC ACGACGGAGACTACAAGGATCATGATATTGATTACAAAG ACGATGACGATAAGATGGCCCCAAAGAAGAAGCGGAAG GTCGGTATCCACGGAGTCCCAGCAGCCGACAAGAAGTAC AGCATCGGCCTGGACATCGGCACCAACTCTGTGGGCTGG GCCGTGATCACCGACGAGTACAAGGTGCCCAGCAAGAAA TTCAAGGTGCTGGGCAACACCGACCGGCACAGCATCAAG AAGAACCTGATCGGAGCCCTGCTGTTCGACAGCGGCGAA ACAGCCGAGGCCACCCGGCTGAAGAGAACCGCCAGAAG AAGATACACCAGACGGAAGAACCGGATCTGCTATCTGCA AGAGATCTTCAGCAACGAGATGGCCAAGGTGGACGACAG CTTCTTCCACAGACTGGAAGAGTCCTTCCTGGTGGAAGAG GATAAGAAGCACGAGCGGCACCCCATCTTCGGCAACATC GTGGACGAGGTGGCCTACCACGAGAAGTACCCCACCATC TACCACCTGAGAAAGAAACTGGTGGACAGCACCGACAAG GCCGACCTGCGGCTGATCTATCTGGCCCTGGCCCACATGA TCAAGTTCCGGGGCCACTTCCTGATCGAGGGCGACCTGA ACCCCGACAACAGCGACGTGGACAAGCTGTTCATCCAGC TGGTGCAGACCTACAACCAGCTGTTCGAGGAAAACCCCA TCAACGCCAGCGGCGTGGACGCCAAGGCCATCCTGTCTG CCAGACTGAGCAAGAGCAGACGGCTGGAAAATCTGATCG CCCAGCTGCCCGGCGAGAAGAAGAATGGCCTGTTCGGAA ACCTGATTGCCCTGAGCCTGGGCCTGACCCCCAACTTCAA GAGCAACTTCGACCTGGCCGAGGATGCCAAACTGCAGCT GAGCAAGGACACCTACGACGACGACCTGGACAACCTGCT |
| | | GGCCCAGATCGGCGACCAGTACGCCGACCTGTTTCTGGC CGCCAAGAACCTGTCCGACGCCATCCTGCTGAGCGACAT CCTGAGAGTGAACACCGAGATCACCAAGGCCCCCCTGAG CGCCTCTATGATCAAGAGATACGACGAGCACCACCAGGA CCTGACCCTGCTGAAAGCTCTCGTGCGGCAGCAGCTGCCT GAGAAGTACAAAGAGATTTTCTTCGACCAGAGCAAGAAC GGCTACGCCGGCTACATTGACGGCGGAGCCAGCCAGGAA GAGTTCTACAAGTTCATCAAGCCCATCCTGGAAAAGATG GACGGCACCGAGGAACTGCTCGTGAAGCTGAACAGAGAG GACCTGCTGCGGAAGCAGCGGACCTTCGACAACGGCAGC ATCCCCCACCAGATCCACCTGGGAGAGCTGCACGCCATT CTGCGGCGGCAGGAAGATTTTTACCCATTCCTGAAGGAC AACCGGGAAAAGATCGAGAAGATCCTGACCTTCCGCATC CCCTACTACGTGGGCCCTCTGGCCAGGGGAAACAGCAGA TTCGCCTGGATGACCAGAAAGAGCGAGGAAACCATCACC CCCTGGAACTTCGAGGAAGTGGTGGACAAGGGCGCTTCC GCCCAGAGCTTCATCGAGCGGATGACCAACTTCGATAAG AACCTGCCCAACGAGAAGGTGCTGCCCAAGCACAGCCTG CTGTACGAGTACTTCACCGTGTATAACGAGCTGACCAAA GTGAAATACGTGACCGAGGGAATGAGAAAGCCCGCCTTC CTGAGCGGCGAGCAGAAAAAGGCCATCGTGGACCTGCTG TTCAAGACCAACCGGAAAGTGACCGTGAAGCAGCTGAAA GAGGACTACTTCAAGAAAATCGAGTGCTTCGACTCCGTG GAAATCTCCGGCGTGGAAGATCGGTTCAACGCCTCCCTG GGCACATACCACGATCTGCTGAAAATTATCAAGGACAAG GACTTCCTGGACAATGAGGAAAACGAGGACATTCTGGAA GATATCGTGCTGACCCTGACACTGTTTGAGGACAGAGAG ATGATCGAGGAACGGCTGAAAACCTATGCCCACCTGTTC GACGACAAAGTGATGAAGCAGCTGAAGCGGCGGAGATA CACCGGCTGGGGCAGGCTGAGCCGGAAGCTGATCAACGG CATCCGGGACAAGCAGTCCGGCAAGACAATCCTGGATTT CCTGAAGTCCGACGGCTTCGCCAACAGAAACTTCATGCA GCTGATCCACGACGACAGCCTGACCTTTAAAGAGGACAT CCAGAAAGCCCAGGTGTCCGGCCAGGGCGATAGCCTGCA CGAGCACATTGCCAATCTGGCCGGCAGCCCCGCCATTAA GAAGGGCATCCTGCAGACAGTGAAGGTGGTGGACGAGCT CGTGAAAGTGATGGGCCGGCACAAGCCCGAGAACATCGT GATCGAAATGGCCAGAGAGAACCAGACCACCCAGAAGG GACAGAAGAACAGCCGCGAGAGAATGAAGCGGATCGAA GAGGGCATCAAAGAGCTGGGCAGCCAGATCCTGAAAGA ACACCCCGTGGAAAACACCCAGCTGCAGAACGAGAAGCT GTACCTGTACTACCTGCAGAATGGGCGGGATATGTACGT GGACCAGGAACTGGACATCAACCGGCTGTCCGACTACGA TGTGGACCATATCGTGCCTCAGAGCTTTCTGAAGGACGAC TCCATCGACAACAAGGTGCTGACCAGAAGCGACAAGAAC CGGGGCAAGAGCGACAACGTGCCCTCCGAAGAGGTCGTG AAGAAGATGAAGAACTACTGGCGGCAGCTGCTGAACGCC AAGCTGATTACCCAGAGAAAGTTCGACAATCTGACCAAG GCCGAGAGAGGCGGCCTGAGCGAACTGGATAAGGCCGG CTTCATCAAGAGACAGCTGGTGGAAACCCGGCAGATCAC AAAGCACGTGGCACAGATCCTGGACTCCCGGATGAACAC TAAGTACGACGAGAATGACAAGCTGATCCGGGAAGTGAA AGTGATCACCCTGAAGTCCAAGCTGGTGTCCGATTTCCGG AAGGATTTCCAGTTTTACAAAGTGCGCGAGATCAACAAC |
| | | TACCACCACGCCCACGACGCCTACCTGAACGCCGTCGTG GGAACCGCCCTGATCAAAAAGTACCCTAAGCTGGAAAGC GAGTTCGTGTACGGCGACTACAAGGTGTACGACGTGCGG AAGATGATCGCCAAGAGCGAGCAGGAAATCGGCAAGGC TACCGCCAAGTACTTCTTCTACAGCAACATCATGAACTTT TTCAAGACCGAGATTACCCTGGCCAACGGCGAGATCCGG AAGCGGCCTCTGATCGAGACAAACGGCGAAACCGGGGA GATCGTGTGGGATAAGGGCCGGGATTTTGCCACCGTGCG GAAAGTGCTGAGCATGCCCCAAGTGAATATCGTGAAAAA GACCGAGGTGCAGACAGGCGGCTTCAGCAAAGAGTCTAT CCTGCCCAAGAGGAACAGCGATAAGCTGATCGCCAGAAA GAAGGACTGGGACCCTAAGAAGTACGGCGGCTTCGACAG CCCCACCGTGGCCTATTCTGTGCTGGTGGTGGCCAAAGTG GAAAAGGGCAAGTCCAAGAAACTGAAGAGTGTGAAAGA GCTGCTGGGGATCACCATCATGGAAAGAAGCAGCTTCGA GAAGAATCCCATCGACTTTCTGGAAGCCAAGGGCTACAA AGAAGTGAAAAAGGACCTGATCATCAAGCTGCCTAAGTA CTCCCTGTTCGAGCTGGAAAACGGCCGGAAGAGAATGCT GGCCTCTGCCGGCGAACTGCAGAAGGGAAACGAACTGGC CCTGCCCTCCAAATATGTGAACTTCCTGTACCTGGCCAGC CACTATGAGAAGCTGAAGGGCTCCCCCGAGGATAATGAG CAGAAACAGCTGTTTGTGGAACAGCACAAGCACTACCTG GACGAGATCATCGAGCAGATCAGCGAGTTCTCCAAGAGA GTGATCCTGGCCGACGCTAATCTGGACAAAGTGCTGTCC GCCTACAACAAGCACCGGGATAAGCCCATCAGAGAGCAG GCCGAGAATATCATCCACCTGTTTACCCTGACCAATCTGG GAGCCCCTGCCGCCTTCAAGTACTTTGACACCACCATCGA CCGGAAGAGGTACACCAGCACCAAAGAGGTGCTGGACGC CACCCTGATCCACCAGAGCATCACCGGCCTGTACGAGAC ACGGATCGACCTGTCTCAGCTGGGAGGCGACAAAAGGCC GGCGGCCACGAAAAAGGCCGGCCAGGCAAAAAAGAAAA AGGAATTCGGCAGTGGAGAGGGCAGAGGAAGTCTGCTAA CATGCGGTGACGTCGAGGAGAATCCTGGCCCAATGACCG AGTACAAGCCCACGGTGCGCCTCGCCACCCGCGACGACG TCCCCAGGGCCGTACGCACCCTCGCCGCCGCGTTCGCCGA CTACCCCGCCACGCGCCACACCGTCGATCCGGACCGCCA CATCGAGCGGGTCACCGAGCTGCAAGAACTCTTCCTCAC GCGCGTCGGGCTCGACATCGGCAAGGTGTGGGTCGCGGA CGACGGCGCCGCGGTGGCGGTCTGGACCACGCCGGAGAG CGTCGAAGCGGGGGCGGTGTTCGCCGAGATCGGCCCGCG CATGGCCGAGTTGAGCGGTTCCCGGCTGGCCGCGCAGCA ACAGATGGAAGGCCTCCTGGCGCCGCACCGGCCCAAGGA GCCCGCGTGGTTCCTGGCCACCGTCGGAGTCTCGCCCGAC CACCAGGGCAAGGGTCTGGGCAGCGCCGTCGTGCTCCCC GGAGTGGAGGCGGCCGAGCGCGCCGGGGTGCCCGCCTTC CTGGAGACCTCCGCGCCCCGCAACCTCCCCTTCTACGAGC GGCTCGGCTTCACCGTCACCGCCGACGTCGAGGTGCCCG AAGGACCGCGCACCTGGTGCATGACCCGCAAGCCCGGTG CCTGAGAATTCTAACTAGAGCTCGCTGATCAGCCTCGACT GTGCCTTCTAGTTGCCAGCCATCTGTTGTTTGCCCCTCCCC CGTGCCTTCCTTGACCCTGGAAGGTGCCACTCCCACTGTC CTTTCCTAATAAAATGAGGAAATTGCATCGCATTGTCTGA GTAGGTGTCATTCTATTCTGGGGGGTGGGGTGGGGCAGG ACAGCAAGGGGGAGGATTGGGAAGAGAATAGCAGGCAT |
| | | GCTGGGGAGCGGCCGCAGGAACCCCTAGTGATGGAGTTG GCCACTCCCTCTCTGCGCGCTCGCTCGCTCACTGAGGCCG GGCGACCAAAGGTCGCCCGACGCCCGGGCTTTGCCCGGG CGGCCTCAGTGAGCGAGCGAGCGCGCAGCTGCCTGCAGG GGCGCCTGATGCGGTATTTTCTCCTTACGCATCTGTGCGG TATTTCACACCGCATACGTCAAAGCAACCATAGTACGCG CCCTGTAGCGGCGCATTAAGCGCGGCGGGTGTGGTGGTT ACGCGCAGCGTGACCGCTACACTTGCCAGCGCCTTAGCG CCCGCTCCTTTCGCTTTCTTCCCTTCCTTTCTCGCCACGTT CGCCGGCTTTCCCCGTCAAGCTCTAAATCGGGGGCTCCCT TTAGGGTTCCGATTTAGTGCTTTACGGCACCTCGACCCCA AAAAACTTGATTTGGGTGATGGTTCACGTAGTGGGCCATC GCCCTGATAGACGGTTTTTCGCCCTTTGACGTTGGAGTCC ACGTTCTTTAATAGTGGACTCTTGTTCCAAACTGGAACAA CACTCAACTCTATCTCGGGCTATTCTTTTGATTTATAAGG GATTTTGCCGATTTCGGTCTATTGGTTAAAAAATGAGCTG ATTTAACAAAAATTTAACGCGAATTTTAACAAAATATTAA CGTTTACAATTTTATGGTGCACTCTCAGTACAATCTGCTC TGATGCCGCATAGTTAAGCCAGCCCCGACACCCGCCAAC ACCCGCTGACGCGCCCTGACGGGCTTGTCTGCTCCCGGCA TCCGCTTACAGACAAGCTGTGACCGTCTCCGGGAGCTGC ATGTGTCAGAGGTTTTCACCGTCATCACCGAAACGCGCG AGACGAAAGGGCCTCGTGATACGCCTATTTTTATAGGTTA ATGTCATGATAATAATGGTTTCTTAGACGTCAGGTGGCAC TTTTCGGGGAAATGTGCGCGGAACCCCTATTTGTTTATTT TTCTAAATACATTCAAATATGTATCCGCTCATGAGACAAT AACCCTGATAAATGCTTCAATAATATTGAAAAAGGAAGA GTATGAGTATTCAACATTTCCGTGTCGCCCTTATTCCCTTT TTTGCGGCATTTTGCCTTCCTGTTTTTGCTCACCCAGAAAC GCTGGTGAAAGTAAAAGATGCTGAAGATCAGTTGGGTGC ACGAGTGGGTTACATCGAACTGGATCTCAACAGCGGTAA GATCCTTGAGAGTTTTCGCCCCGAAGAACGTTTTCCAATG ATGAGCACTTTTAAAGTTCTGCTATGTGGCGCGGTATTAT CCCGTATTGACGCCGGGCAAGAGCAACTCGGTCGCCGCA TACACTATTCTCAGAATGACTTGGTTGAGTACTCACCAGT CACAGAAAAGCATCTTACGGATGGCATGACAGTAAGAGA ATTATGCAGTGCTGCCATAACCATGAGTGATAACACTGC GGCCAACTTACTTCTGACAACGATCGGAGGACCGAAGGA GCTAACCGCTTTTTTGCACAACATGGGGGATCATGTAACT CGCCTTGATCGTTGGGAACCGGAGCTGAATGAAGCCATA CCAAACGACGAGCGTGACACCACGATGCCTGTAGCAATG GCAACAACGTTGCGCAAACTATTAACTGGCGAACTACTT ACTCTAGCTTCCCGGCAACAATTAATAGACTGGATGGAG GCGGATAAAGTTGCAGGACCACTTCTGCGCTCGGCCCTTC CGGCTGGCTGGTTTATTGCTGATAAATCTGGAGCCGGTGA GCGTGGAAGCCGCGGTATCATTGCAGCACTGGGGCCAGA TGGTAAGCCCTCCCGTATCGTAGTTATCTACACGACGGGG AGTCAGGCAACTATGGATGAACGAAATAGACAGATCGCT GAGATAGGTGCCTCACTGATTAAGCATTGGTAACTGTCA GACCAAGTTTACTCATATATACTTTAGATTGATTTAAAAC TTCATTTTTAATTTAAAAGGATCTAGGTGAAGATCCTTTT TGATAATCTCATGACCAAAATCCCTTAACGTGAGTTTTCG TTCCACTGAGCGTCAGACCCCGTAGAAAAGATCAAAGGA TCTTCTTGAGATCCTTTTTTTCTGCGCGTAATCTGCTGCTT |
| | | GCAAACAAAAAAACCACCGCTACCAGCGGTGGTTTGTTT GCCGGATCAAGAGCTACCAACTCTTTTTCCGAAGGTAACT GGCTTCAGCAGAGCGCAGATACCAAATACTGTTCTTCTAG TGTAGCCGTAGTTAGGCCACCACTTCAAGAACTCTGTAGC ACCGCCTACATACCTCGCTCTGCTAATCCTGTTACCAGTG GCTGCTGCCAGTGGCGATAAGTCGTGTCTTACCGGGTTGG ACTCAAGACGATAGTTACCGGATAAGGCGCAGCGGTCGG GCTGAACGGGGGGTTCGTGCACACAGCCCAGCTTGGAGC GAACGACCTACACCGAACTGAGATACCTACAGCGTGAGC TATGAGAAAGCGCCACGCTTCCCGAAGGGAGAAAGGCGG ACAGGTATCCGGTAAGCGGCAGGGTCGGAACAGGAGAG CGCACGAGGGAGCTTCCAGGGGGAAACGCCTGGTATCTT TATAGTCCTGTCGGGTTTCGCCACCTCTGACTTGAGCGTC GATTTTTGTGATGCTCGTCAGGGGGGCGGAGCCTATGGA AAAACGCCAGCAACGCGGCCTTTTTACGGTTCCTGGCCTT TTGCTGGCCTTTTGCTCACATGT |
| 2 | Donor vector | TGCAGCTCTGGCCCGTGTCTCAAAATCTCTGATGTTACAT TGCACAAGATAAAAATATATCATCATGAACAATAAAACT GTCTGCTTACATAAACAGTAATACAAGGGGTGTTATGAG CCATATTCAACGGGAAACGTCGAGGCCGCGATTAAATTC CAACATGGATGCTGATTTATATGGGTATAAATGGGCTCGC GATAATGTCGGGCAATCAGGTGCGACAATCTATCGCTTGT ATGGGAAGCCCGATGCGCCAGAGTTGTTTCTGAAACATG GCAAAGGTAGCGTTGCCAATGATGTTACAGATGAGATGG TCAGACTAAACTGGCTGACGGAATTTATGCCTCTTCCGAC CATCAAGCATTTTATCCGTACTCCTGATGATGCATGGTTA CTCACCACTGCGATCCCCGGAAAAACAGCATTCCAGGTA TTAGAAGAATATCCTGATTCAGGTGAAAATATTGTTGATG CGCTGGCAGTGTTCCTGCGCCGGTTGCATTCGATTCCTGT TTGTAATTGTCCTTTTAACAGCGATCGCGTATTTCGTCTC GCTCAGGCGCAATCACGAATGAATAACGGTTTGGTTGAT GCGAGTGATTTTGATGACGAGCGTAATGGCTGGCCTGTTG AACAAGTCTGGAAAGAAATGCATAAACTTTTGCCATTCTC ACCGGATTCAGTCGTCACTCATGGTGATTTCTCACTTGAT AACCTTATTTTTGACGAGGGGAAATTAATAGGTTGTATTG ATGTTGGACGAGTCGGAATCGCAGACCGATACCAGGATC TTGCCATCCTATGGAACTGCCTCGGTGAGTTTTCTCCTTC ATTACAGAAACGGCTTTTTCAAAAATATGGTATTGATAAT CCTGATATGAATAAATTGCAGTTTCATTTGATGCTCGATG AGTTTTTCTAATCAGAATTGGTTAATTGGTTGTAACATTA TTCAGATTGGGCTTGATTTAAAACTTCATTTTTAATTTAA AAGGATCTAGGTGAAGATCCTTTTTGATAATCTCATGACC AAAATCCCTTAACGTGAGTTTTCGTTCCACTGAGCGTCAG ACCCCGTAGAAAAGATCAAAGGATCTTCTTGAGATCCTTT TTTTCTGCGCGTAATCTGCTGCTTGCAAACAAAAAAACCA CCGCTACCAGCGGTGGTTTGTTTGCCGGATCAAGAGCTAC CAACTCTTTTTCCGAAGGTAACTGGCTTCAGCAGAGCGCA GATACCAAATACTGTTCTTCTAGTGTAGCCGTAGTTAGGC CACCACTTCAAGAACTCTGTAGCACCGCCTACATACCTCG CTCTGCTAATCCTGTTACCAGTGGCTGCTGCCAGTGGCGA TAAGTCGTGTCTTACCGGGTTGGACTCAAGACGATAGTTA CCGGATAAGGCGCAGCGGTCGGGCTGAACGGGGGGTTCG TGCACACAGCCCAGCTTGGAGCGAACGACCTACACCGAA CTGAGATACCTACAGCGTGAGCTATGAGAAAGCGCCACG |
| | | CTTCCCGAAGGGAGAAAGGCGGACAGGTATCCGGTAAGC GGCAGGGTCGGAACAGGAGAGCGCACGAGGGAGCTTCC AGGGGGAAACGCCTGGTATCTTTATAGTCCTGTCGGGTTT CGCCACCTCTGACTTGAGCGTCGATTTTTGTGATGCTCGT CAGGGGGGCGGAGCCTATGGAAAAACGCCAGCAACGCG GCCTTTTTACGGTTCCTGGCCTTTTGCTGGCCTTTTGCTCA CATGTTCTTTCCTGCGTTATCCCCTGATTCTGTGGATAACC GTATTACCGCCTTTGAGTGAGCTGATACCGCTCGCCGCAG CCGAACGACCGAGCGCAGCGAGTCAGTGAGCGAGGAAG CGGAAGAGCGCCCAATACGCAAACCGCCTCTCCCCGCGC GTTGGCCGATTCATTAATGCAGCTGGCACGACAGGTTTCC CGACTGGAAAGCGGGCAGTGAGCGCAACGCAATTAATGT GAGTTAGCTCACTCATTAGGCACCCCAGGCTTTACACTTT ATGCTTCCGGCTCGTATGTTGTGTGGAATTGTGAGCGGAT AACAATTTCACACAGGAAACAGCTATGACCATGATTACG CCGCGGCCGCTGCTTTCTCTGACCAGCATTCTCTCCCCTG GGCCTGTGCCGCTTTCTGTCTGCAGCTTGTGGCCTGGGTC ACCTCTACGGCTGGCCCAGATCCTTCCCTGCCGCCTCCTT CAGGTTCCGTCTTCCTCCACTCCCTCTTCCCCTTGCTCTCT GCTGTGTTGCTGCCCAAGGATGCTCTTTCCGGAGCACTTC CTTCTCGGCGCTGCACCACGTGATGTCCTCTGAGCGGATC CTCCCCGTGTCTGGGTCCTCTCCGGGCATCTCTCCTCCCTC ACCCAACCCCATGCCGTCTTCACTCGCTGGGTTCCCTTTT CCTTCTCCTTCTGGGGCCTGTGCCATCTCTCGTTTCTTAGG ATGGCCTTCTCCGACGGATGTCTCCCTTGCGTCCCGCCTC CCCTTCTTGTAGGCCTGCATCATCACCGTTTTTCTGGACA ACCCCAAAGTACCCCGTCTCCCTGGCTTTAGCCACCTCTC CATCCTCTTGCTTTCTTTGCCTGGACACCCCGTTCTCCTGT GGATTCGGGTCACCTCTCACTCCTTTCATTTGGGCAGCTC CCCTACCCCCCTTACCTCTCTAGTCTGTGCTAGCTCTTCCA GCCCCCTGTCATGGCATCTTCCAGGGGTCCGAGAGCTCAG CTAGTCTTCTTCCTCCAACCCGGGCCCCTATGTCCACTTC AGGACAGCATGTTTGCTGCCTCCAGGGATCCTGTGTCCCC GAGCTGGGACCACCTTATATTCCCAGGGCCGGTTAATGTG GCTCTGGTTCTGGGTACTTTTATCTGTCCCCTCCACCCCAC AGTGGGGCGCGGCCGCATAACTTCGTATAGCATACATTA TACGAAGTTATCTGACCTCTTCTCTTCCTCCCACAGGGCC TCGAGAGATCTGGCAGCGGAGAGGGCAGAGGAAGTCTTC TAACATGCGGTGACGTGGAGGAGAATCCCGGCCCTAGGA TGACCGAGTACAAGCCCACGGTGCGCCTCGCCACCCGCG ACGACGTCCCCAGGGCCGTACGCACCCTCGCCGCCGCGT TCGCCGACTACCCCGCCACGCGCCACACCGTCGATCCGG ACCGCCACATCGAGCGGGTCACCGAGCTGCAAGAACTCT TCCTCACGCGCGTCGGGCTCGACATCGGCAAGGTGTGGG TCGCGGACGACGGCGCCGCGGTGGCGGTCTGGACCACGC CGGAGAGCGTCGAAGCGGGGGCGGTGTTCGCCGAGATCG GCCCGCGCATGGCCGAGTTGAGCGGTTCCCGGCTGGCCG CGCAGCAACAGATGGAAGGCCTCCTGGCGCCGCACCGGC CCAAGGAGCCCGCGTGGTTCCTGGCCACCGTCGGCGTCTC GCCCGACCACCAGGGCAAGGGTCTGGGCAGCGCCGTCGT GCTCCCCGGAGTGGAGGCGGCCGAGCGCGCCGGGGTGCC CGCCTTCCTGGAGACCTCCGCGCCCCGCAACCTCCCCTTC TACGAGCGGCTCGGCTTCACCGTCACCGCCGACGTCGAG GTGCCCGAAGGACCGCGCACCTGGTGCATGACCCGCAAG |
| | | CCCGGTGCCTGACTGTGCCTTCTAGTTGCCAGCCATCTGT TGTTTGCCCCTCCCCCGTGCCTTCCTTGACCCTGGAAGGT GCCACTCCCACTGTCCTTTCCTAATAAAATGAGGAAATTG CATCGCATTGTCTGAGTAGGTGTCATTCTATTCTGGGGGG TGGGGTGGGGCAGGACAGCAAGGGGGAGGATTGGGAAG ACAATAGCAGGCATGCTGGGGATGCGGTGGGCTCTATGG ATAACTTCGTATAGCATACATTATACGAAGTTATGAGCTC ACGGGGACAGCCCCCCCCCAAAGCCCCCAGGGATGTAAT TACGTCCCTCCCCCGCTAGGGGGCAGCAGCGAGCCGCCC GGGGCTCCGCTCCGGTCCGGCGCTCCCCCCGCATCCCCGA GCCGGCAGCGTGCGGGGACAGCCCGGGCACGGGGAAGG TGGCACGGGATCGCTTTCCTCTGAACGCTTCTCGCTGCTC TTTGAGCCTGCAGACACCTGGGGGGATACGGGGAAAAAG CTTTAGGCTGAAAGAGAGATTTAGAATGACAGAATCATA GAACGGCCTGGGTTGCAAAGGAGCACAGTGCTCATCCAG ATCCAACCCCCTGCTATGTGCAGGGTCATCAACCAGCAG CCCAGGCTGCCCAGAGCCACATCCAGCCTGGCCTTGAAT GCCTGCAGGGATGGGGCATCCACAGCCTCCTTGGGCAAC CTGTTCAGTGCGTCACCACCCTCTGGGGGAAAAACTGCCT CCTCATATCCAACCCAAACCTCCCCTGTCTCAGTGTAAAG CCATTCCCCCTTGTCCTATCAAGGGGGAGTTTGCTGTGAC ATTGTTGGTCTGGGGTGACACATGTTTGCCAATTCAGTGC ATCACGGAGAGGCAGATCTTGGGGATAAGGAAGTGCAGG ACAGCATGGACGTGGGACATGCAGGTGTTGAGGGCTCTG GGACACTCTCCAAGTCACAGCGTTCAGAACAGCCTTAAG GATAAGAAGATAGGATAGAAGGACAAAGAGCAAGTTAA AACCCAGCATGGAGAGGAGCACAAAAAGGCCACAGACA CTGCTGGTCCCTGTGTCTGAGCCTGCATGTTTGATGGTGT CTGGATGCAAGCAGAAGGGGTGGAAGAGCTTGCCTGGAG AGATACAGCTGGGTCAGTAGGACTGGGACAGGCAGCTGG AGAATTGCCATGTAGATGTTCATACAATCGTCAAATCATG AAGGCTGGAAAAGCCCTCCAAGATCCCCAAGACCAACCC CAACCCACCCACCGTGCCCACTGGCCATGTCCCTCAGTGC CACATCCCCACAGTTCTTCATCACCTCCAGGGACGGTGAC CCCCCCACCTCCGTGGGCAGCTGTGCCACTGCAGCACCGC TCTTTGGAGAAGGTAAATCTTGCTAAATCCAGCCCGACCC TCCCCTGGCACAACGTAAGGCCATTATCTCTCATCCAACT CCAGGACGGAGTCAGTGAGAATATTGGCGCGCCGAATTC TCGCGACGATCGGTCGACATAACTTCGTATAGGATACTTT ATACGAAGTTATCAATTCAGGCTAGTTATTAATAGTAATC AATTACGGGGTCATTAGTTCATAGCCCATATATGGAGTTC CGCGTTACATAACTTACGGTAAATGGCCCGCCTGGCTGAC CGCCCAACGACCCCCGCCCATTGACGTCAATAATGACGT ATGTTCCCATAGTAACGCCAATAGGGACTTTCCATTGACG TCAATGGGTGGAGTATTTACGGTAAACTGCCCACTTGGCA GTACATCAAGTGTATCATATGCCAAGTACGCCCCCTATTG ACGTCAATGACGGTAAATGGCCCGCCTGGCATTATGCCC AGTACATGACCTTATGGGACTTTCCTACTTGGCAGTACAT CTACGTATTAGTCATCGCTATTACCATGGTCGAGGTGAGC CCCACGTTCTGCTTCACTCTCCCCATCTCCCCCCCCTCCCC ACCCCCAATTTTGTATTTATTTATTTTTTAATTATTTTGTG CAGCGATGGGGGCGGGGGGGGGGGGGGGGCGCGCGCCA GGCGGGGCGGGGCGGGGCGAGGGGCGGGGCGGGGCGAG GCGGAGAGGTGCGGCGGCAGCCAATCAGAGCGGCGCGCT |
| | | CCGAAAGTTTCCTTTTATGGCGAGGCGGCGGCGGCGGCG GCCCTATAAAAAGCGAAGCGCGCGGCGGGCGGGGAGTC GCTGCGACGCTGCCTTCGCCCCGTGCCCCGCTCCGCCGCC GCCTCGCGCCGCCCGCCCCGGCTCTGACTGACCGCGTTAC TCCCACAGGTGAGCGGGCGGGACGGCCCTTCTCCTCCGG GCTGTAATTAGCGCTTGGTTTAATGACGGCTTGTTTCTTTT CTGTGGCTGCGTGAAAGCCTTGAGGGGCTCCGGGAGGGC CCTTTGTGCGGGGGGAGCGGCTCGGGGGGTGCGTGCGTG TGTGTGTGCGTGGGGAGCGCCGCGTGCGGCTCCGCGCTG CCCGGCGGCTGTGAGCGCTGCGGGCGCGGCGCGGGGCTT TGTGCGCTCCGCAGTGTGCGCGAGGGGAGCGCGGCCGGG GGCGGTGCCCCGCGGTGCGGGGGGGGCTGCGAGGGGAA CAAAGGCTGCGTGCGGGGTGTGTGCGTGGGGGGGTGAGC AGGGGGTGTGGGCGCGTCGGTCGGGCTGCAACCCCCCCT GCACCCCCCTCCCCGAGTTGCTGAGCACGGCCCGGCTTCG GGTGCGGGGCTCCGTACGGGGCGTGGCGCGGGGCTCGCC GTGCCGGGCGGGGGGTGGCGGCAGGTGGGGGTGCCGGG CGGGGCGGGGCCGCCTCGGGCCGGGGAGGGCTCGGGGG AGGGGCGCGGCGGCCCCCGGAGCGCCGGCGGCTGTCGAG GCGCGGCGAGCCGCAGCCATTGCCTTTTATGGTAATCGTG CGAGAGGGCGCAGGGACTTCCTTTGTCCCAAATCTGTGC GGAGCCGAAATCTGGGAGGCGCCGCCGCACCCCCTCTAG CGGGCGCGGGGCGAAGCGGTGCGGCGCCGGCAGGAAGG AAATGGGCGGGGAGGGCCTTCGTGCGTCGCCGCGCCGCC GTCCCCTTCTCCCTCTCCAGCCTCGGGGCTGTCCGCGGGG GGACGGCTGCCTTCGGGGGGGACGGGGCAGGGCGGGGTT CGGCTTCTGGCGTGTGACCGGCGGCTCTAGAGCCTCTGCT AACCATGTTCATGCCTTCTTCTTTTTCCTACAGCTCCTGGG CAACGTGCTGGTTATTGTGCTGTCTCATCATTTTGGCAAA GAATTGTGTACAACCATGGAGAGCGACGAGAGCGGCCTG CCCGCCATGGAGATCGAGTGCCGCATCACCGGCACCCTG AACGGCGTGGAGTTCGAGCTGGTGGGCGGCGGAGAGGGC ACCCCCAAGCAGGGCCGCATGACCAACAAGATGAAGAGC ACCAAAGGCGCCCTGACCTTCAGCCCCTACCTGCTGAGCC ACGTGATGGGCTACGGCTTCTACCACTTCGGCACCTACCC CAGCGGCTACGAGAACCCCTTCCTGCACGCCATCAACAA CGGCGGCTACACCAACACCCGCATCGAGAAGTACGAGGA CGGCGGCGTGCTGCACGTGAGCTTCAGCTACCGCTACGA GGCCGGCCGCGTGATCGGCGACTTCAAGGTGGTGGGCAC CGGCTTCCCCGAGGACAGCGTGATCTTCACCGACAAGAT CATCCGCAGCAACGCCACCGTGGAGCACCTGCACCCCAT GGGCGATAACGTGCTGGTGGGCAGCTTCGCCCGCACCTT CAGCCTGCGCGACGGCGGCTACTACAGCTTCGTGGTGGA CAGCCACATGCACTTCAAGAGCGCCATCCACCCCAGCAT CCTGCAGAACGGGGGCCCCATGTTCGCCTTCCGCCGCGTG GAGGAGCTGCACAGCAACACCGAGCTGGGCATCGTGGAG TACCAGCACGCCTTCAAGACCCCCATCGCCTTCGCCAGAT CCCGCGCTCAGTCGTCCAATTCTGCCGTGGACGGCACCGC CGGACCCGGCTCCACCGGATCTCGCTAAACGCGTGAATT CACTCCTCAGGTGCAGGCTGCCTATCAGAAGGTGGTGGC TGGTGTGGCCAATGCCCTGGCTCACAAATACCACTGAGA TCTTTTTCCCTCTGCCAAAAATTATGGGGACATCATGAAG CCCCTTGAGCATCTGACTTCTGGCTAATAAAGGAAATTTA TTTTCATTGCAATAGTGTGTTGGAATTTTTTGTGTCTCTCA |
| | | CTCGGAAGGACATATGGGAGGGCAAATCATTTAAAACAT CAGAATGAGTATTTGGTTTAGAGTTTGGCAACATATGCCC ATATGCTGGCTGCCATGAACAAAGGTTGGCTATAAAGAG GTCATCAGTATATGAAACAGCCCCCTGCTGTCCATTCCTT ATTCCATAGAAAAGCCTTGACTTGAGGTTAGATTTTTTTT ATATTTTGTTTTGTGTTATTTTTTTCTTTAACATCCCTAAA ATTTTCCTTACATGTTTTACTAGCCAGATTTTTCCTCCTCT CCTGACTACTCCCAGTCATAGCTGTCCCTCTTCTCTTATG GAGATCACCGGTATAACTTCGTATAATGTATACTATACGA AGTTATGGTACCTTAATTAAGTTAACATGCATAGTACTGA GCTCACGGGGACAGCCCCCCCCCAAAGCCCCCAGGGATG TAATTACGTCCCTCCCCCGCTAGGGGGCAGCAGCGAGCC GCCCGGGGCTCCGCTCCGGTCCGGCGCTCCCCCCGCATCC CCGAGCCGGCAGCGTGCGGGGACAGCCCGGGCACGGGG AAGGTGGCACGGGATCGCTTTCCTCTGAACGCTTCTCGCT GCTCTTTGAGCCTGCAGACACCTGGGGGGATACGGGGAA AAAGCTTTAGGCTGAAAGAGAGATTTAGAATGACAGAAT CATAGAACGGCCTGGGTTGCAAAGGAGCACAGTGCTCAT CCAGATCCAACCCCCTGCTATGTGCAGGGTCATCAACCA GCAGCCCAGGCTGCCCAGAGCCACATCCAGCCTGGCCTT GAATGCCTGCAGGGATGGGGCATCCACAGCCTCCTTGGG CAACCTGTTCAGTGCGTCACCACCCTCTGGGGGAAAAAC TGCCTCCTCATATCCAACCCAAACCTCCCCTGTCTCAGTG TAAAGCCATTCCCCCTTGTCCTATCAAGGGGGAGTTTGCT GTGACATTGTTGGTCTGGGGTGACACATGTTTGCCAATTC AGTGCATCACGGAGAGGCAGATCTTGGGGATAAGGAAGT GCAGGACAGCATGGACGTGGGACATGCAGGTGTTGAGGG CTCTGGGACACTCTCCAAGTCACAGCGTTCAGAACAGCCT TAAGGATAAGAAGATAGGATAGAAGGACAAAGAGCAAG TTAAAACCCAGCATGGAGAGGAGCACAAAAAGGCCACA GACACTGCTGGTCCCTGTGTCTGAGCCTGCATGTTTGATG GTGTCTGGATGCAAGCAGAAGGGGTGGAAGAGCTTGCCT GGAGAGATACAGCTGGGTCAGTAGGACTGGGACAGGCA GCTGGAGAATTGCCATGTAGATGTTCATACAATCGTCAA ATCATGAAGGCTGGAAAAGCCCTCCAAGATCCCCAAGAC CAACCCCAACCCACCCACCGTGCCCACTGGCCATGTCCCT CAGTGCCACATCCCCACAGTTCTTCATCACCTCCAGGGAC GGTGACCCCCCCACCTCCGTGGGCAGCTGTGCCACTGCA GCACCGCTCTTTGGAGAAGGTAAATCTTGCTAAATCCAGC CCGACCCTCCCCTGGCACAACGTAAGGCCATTATCTCTCA TCCAACTCCAGGACGGAGTCAGTGAGAATATTGTTTAAA CACTAGGGACAGGATTGGTGACAGAAAAGCCCCATCCTT AGGCCTCCTCCTTCCTAGTCTCCTGATATTGGGTCTAACC CCCACCTCCTGTTAGGCAGATTCCTTATCTGGTGACACAC CCCCATTTCCTGGAGCCATCTCTCTCCTTGCCAGAACCTC TAAGGTTTGCTTACGATGGAGCCAGAGAGGATCCTGGGA GGGAGAGCTTGGCAGGGGGTGGGAGGGAAGGGGGGGAT GCGTGACCTGCCCGGTTCTCAGTGGCCACCCTGCGCTACC CTCTCCCAGAACCTGAGCTGCTCTGACGCGGCTGTCTGGT GCGTTTCACTGATCCTGGTGCTGCAGCTTCCTTACACTTC CCAAGAGGAGAAGCAGTTTGGAAAAACAAAATCAGAAT AAGTTGGTCCTGAGTTCTAACTTTGGCTCTTCACCTTTCTA GTCCCCAATTTATATTGTTCCTCCGTGCGTCAGTTTTACCT GTGAGATAAGGCCAGTAGCCAGCCCCGTCCTGGCAGGGC |
| | | TGTGGTGAGGAGGGGGGTGTCCGTGTGGAAAACTCCCTT TGTGAGAATGGTGCGTCCTAGGTGTTCACCAGGTCGTGGC CGCCTCTACTCCCTTTCTCTTTCTCCATCCTTCTTTCCTTA AAGAGTCCCCAGTGCTATCTGGGACATATTCCTCCGCCCA GAGCAGGGTCCCGCTTCCCTAAGGCCCTGCTCTGGGCTTC TGGGTTTGAGTCCTTGGCAAGCCCAGGAGAGGCGCTCAG GCTTCCCTGTCCCCCTTCCTCGTCCACCATCTCATGCCCCT GGCTCTCCTGCCCCTTCCCTACAGGGGTTCCTGGCTCTGC TCTGTTTAAACACTGGCCGTCGTTTTACAACGTCGTGACT GGGAAAACCCTGGCGTTACCCAACTTAATCGCCTTGCAG CACATCCCCCTTTCGCCAGCTGGCGTAATAGCGAAGAGG CCCGCACCGATCGCCCTTCCCAACAGTTGCGCAGCCTGAA TGGCGAATGGCGCCTGATGCGGTATTTTCTCCTTACGCAT CTGTGCGGTATTTCACACCGCATATGGTGCACTCTCAGTA CAATCTGCTCTGATGCCGCATAGTTAAGCCAGCCCCGACA CCCGCCAACACCCGCTGACGCGCCCTGACGGGCTTGTCTG CTCCCGGCATCCGCTTACAGACAAGCTGTGACCGTCTCCG GGAGCTGCATGTGTCAGAGGTTTTCACCGTCATCACCGAA ACGCGCGA |

### [Example 2]

### Selection of genomic safe harbor candidates

GSH candidates were selected based on the following requirements: cancer-related genes, miRNAs, positions more than 300 kb away from functional small RNA, positions more than 50 kb away from the 5' end of the gene, positions more than 50 kb away from the origin of replication, positions more than 50 kb away from ultra-conserved regions, positions with low transcriptional activity, positions not comprised in copy number variation regions, positions comprised in open chromatin regions, sequences with only one copy on human chromosomes, and sequences without TTAA sequences (transposons).

Then, as shown in Table 1, three GSH candidate regions and the corresponding gRNA sequences were acquired using CRISPick, DeepSpCas9, and RGEN as gRNA derivation tools. CRISPick (Broad Institute) indicates how specifically gRNA can react to complementary positions on the genome based on on-target scores. DeepSpCas9 indicates the genome editing efficiency by gRNA based on indel frequency scores. RGEN indicates whether or not there is a mismatch between the chromosome and gRNA.

**[Table 2]**

| SEQ ID NO: | Name | Chromosome | Start | End | gRNA sequence |
|---|---|---|---|---|---|
| 3 | GSH1 | 9 | 24894446 | 24894525 | CTTCAGTGCTCTACTCTTGT |
| 4 | GSH2 | 3 | 9064276 | 9064355 | GTAAGCAAACCACAACGTCT |
| 5 | GSH3 | 4 | 120174229 | 120174308 | TCTGAGACAGCTAATATCAT |

### [Example 3]

### Production of gRNA/Cas9 expression vector targeting AAVS1 and genomic safe harbors

The gRNA insert targeting AAVS1, GSH1, GSH2, and GSH3 was introduced into the gRNA/Cas9 expression vector (px459) as follows.

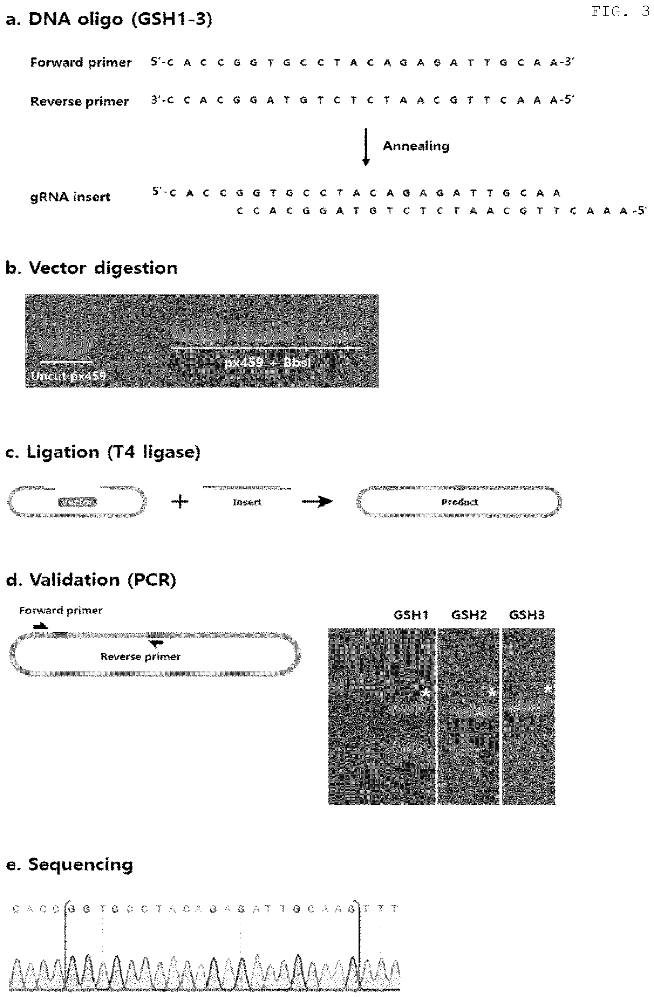

DNA oligos based on the gRNA insert sequence targeting AAVS1, GSH1, GSH2, and GSH3 were synthesized. Then, the gRNA/Cas9 expression vector was cleaved by treatment with the restriction enzyme BbsI, and the synthesized DNA oligo was introduced into the cleaved and linearized gRNA/Cas9 expression vector, followed by ligation. Whether or not the gRNA was introduced by PCR was validated and the gRNA sequence introduced into the vector was validated by Sanger sequencing. A schematic diagram of the process is shown in FIG. 3.

**[Table 3]**

| SEQ ID NO: | Name | Base sequence |
|---|---|---|
| 6 | GSH1-primerF | CACCGTTCAGTGCTCTACTCTTGT |
| 7 | GSH1-primerR | AAACACAAGAGTAGAGCACTGAAC |
| 8 | GSH2-primerF | CACCGTAAGCAAACCACAACGTCT |
| 9 | GSH2-primerR | AAACAGACGTTGTGGTTTGCTTAC |
| 10 | GSH3-primerF | CACCGTCTGAGACAGCTAATATCA |
| 11 | GSH3-primerR | AAACTGATATTAGCTGTCTCAGAC |
| 12 | AAVS1-primerF | ACCGTCCCCTCCACCCCACAGTG |
| 13 | AAVS1-primerR | AACCACTGTGGGGTGGAGGGGAC |

### [Example 4]

### Production of donor vector

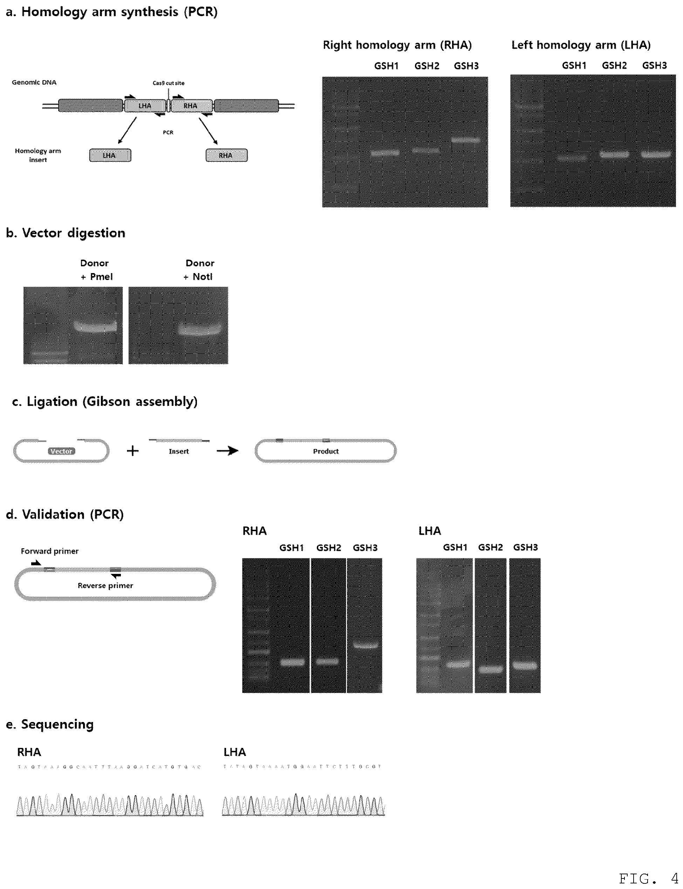

To promote efficient HDR, a homologous sequence (≥800 kb) depending on the target was introduced into the donor vector as follows. About 800 kb upstream and downstream of the position where DSB is caused by the CRISPR-Cas9 system were referred to as a "left homology arm" and a "right homology arm", respectively. Then, the left homology sequence and the right homology sequence of SEQ ID NOs: 14 to 21 according to the location in the genome of AAVS1, GSH1, GSH2, and GSH3 were synthesized based on the genome obtained from human adipose stem cells through PCR. Then, the donor vector was cleaved by treatment with restriction enzymes PmeI and NotI. The homologous sequence was introduced into the donor vector linearized by cleaving using Gibson assembly master mix (NEB), the introduction was validated by PCR, and the homology DNA sequence introduced into the vector was validated by Sanger sequencing. As a result of the process, donor vectors of SEQ ID NOs: 22 to 25 were obtained and a schematic diagram of the process is shown in FIG. 4.

**[Table 4]**

| SEQ ID NO: | Name | Base sequence |
|---|---|---|
| 14 | GSH1-RHA | TGTGGGAAGCTTCTTTCCAACTTGGTTCCTGTCCATACC CACGTAAAATATTAAAGAAAGAAGGATAGAAATATAGA TTAAAAGAAGACCTGAAGTCGAAGGAAGGGACTAATTG TTGAGAGATTTGAAATGCAACCAATTTTCCATCAGATGT TTTATCGGTACTCTGTAGTTCTATTCCGTCCCAGTTTTCC ATAAATTTATAGATAAAAAGAATATTTTATATTGCATTT TGTATGCATATATGCATACATACATATCACTTAAATATA TATATATATAAAATTGCATGTTATGCACACTTTAAAATC TTTAAAATATTGAATGCTTAATTACTTAGGACAAAATAG CTTACATAGGAAACAATTATTTCGGACAGAATAATTTAG GAGACAATAGCATTTTTGTGCTGTTGCAGAGATGATAA AGAACTTCTGGTTTTATATGTAACTCTAAAACTGCAGGA TAAATAGGTACGTAATACAAATGGCAGTTGCCTCACTG CAATTTTCTGAGCTGTGTTGCTAGTCTGACACTCCTGAA AATTAATATTTCAGACATCTCTGTAATTGTCCAGAGGGC CTGGAGTGCCTTCAAATTATATTCCCCCTCTATTTTAATC AACCATGTTAGTAATTTACTTTTCATTAGTTTATTGATAA TTCTATAGGGAAAGTTTGCTAATCACTGTGTAAAATATA ATTTTCAAACAGCCAAAATAACTGCCATAAAATATGGA AACTGTCTTAGTTTGTTTTCTCTTGCTATGAAAGAATAC CACAGACTGGGTAATGTATAGAGAAGAG |
| 15 | GSH1-LHA | GCAAATAGCAAGTCTTCTCTGGTTCTTTGAGCTCTGTTTT TGATGACTTCACCCTGGAGGTAACAGCTTTCCTAAGGCC TGACATGCAGGCCTGCTGCATTGCAAACTCCCTCTGCTC ATTTTTGGCAGGTTGTGCTGATTTATGGGGCTATTTGAT TTATTTAATGTACCATGCCTTTGATTGCATATACTCTATA GCAGTCACTCAAAATGCAAACAAACACATCATATGTTC TTTAATTACTCCCATCCCCCTTATTATCAATCACTGCCAA TATCAGCAGAATGGCTGTGATTCATGCTATAAAATTAAG GTCAGCAAACTTGGGTTTTGCAGAAGTGAATAGCAGCA ATTCTGTTTATATTCTTTAATGTTTCAATTCTAAAACTCA TTATCAGAAAAATGGTATTTCAGATCAGTCTTGTTAGAA GAATGGGATGGGAAAAAAGATATAACAGTTGGGCAAA GAGAAGGAAAGCAAATGTAGCACTAGGGAAAAAGTTGT AGAAGAAAGTAAAAAAAAAAATAAAGATGAGAAGATA GTAGCTCTTAAGGATATTCATTCTGACTTATGAAAGACT GTAATGTTTAGCACACACAGACACATACACAAACACAT |
| | | GAACTTGTAAAATTCTCAAAGGTCCAGTTTTTATTCAAA ATTCCTATACTTCAACTTCTTGAATCTCACTCCTTTTTTT TTCAAGTTCCTCATTTCTCTCCCTTAAAAAATGGATGCT AACCATTTTAAGCTTAGTAATTTTGTACATGCTTTTCTGG AAATACTACAAATATGATCAATGTCTGTGATCTCTTCAG TGCTCTACTCT |
| 16 | GSH2-RHA | TCTAGGCCAAATCTGGCCTCCCAATTGTTTTATAAATAA AATTTTATTTAAACGTAACCACTCCCATTCATTTGCTTGT CTCTGGTTGTTTTTGCAATACAACAGTAGGGTTAGGTAG ATAATACAGGCTGTGTGTCACATGATGAAGCCAAAAAC ATTTGCTCTCTGGCTCTTTACAGAATGAATTTGCTTATAT TTCTTCTAGGATGTCAATTTGTCTGCTTAATTCACAGAA GGACAAATTTTAACATAACTTACTTCTATGGCCTGAATG TTTGCGTACACTGAAAATTGATTTGTTGAAATCGATCCC CAATTTGATAGTATTAGGAGGTAGGGCTTTGGAGATGT GATTAGATCATGAGGGTGTAACCTTTATTAATGGGATTA GTGCTTTTATATGGGGTTGAAGAGACCCGAGCTCCCCTT CCACCTTGTGAGGATGCAGAAAGAAGATACATTCTGTG AACTGGAAAGCCGGCCCTCACTAGACACCAAATATGCC AGTATTTTGATCTTGGGCTTCCCAGCCTTCAGAACTTAC AGCAGTCCAGAAAGATTAAGACACCCACCTGAAATGTT AATTTTGTCCTAAAATAATCTTGCATTTAAGATCTGAAA TATGACCTAAGTATTATCTGAGGAGTCAATGCTGATTTA TTGGTGGTGGATACTGTGATTCTGTATTAAGAATGGATC TGAAGCCACATAAGATAACTGTGATTACTTAGTGCTATT TTCAATGGCTGTGAGAAGACAACCTTGTGACCAGAATTT GAAACCCTGCATAAACAAAACTCAACATGTTTTGAAGG CCTTTACACTCTTCCTCTCAGG |
| 17 | GSH2-LHA | CAGACACATGTTCCCCAGAAGAGGATTTGCCATTCTGAC AAATCTCCATCCAAGGAATGGGAAATTCATTATCCCTTC CACTAGAATTAAACTGTGAAATTATTTATGTAGGAAGA AGTAGTAGTAGAAAAGTTATGTTTGTCTTCTTCACTATA AGAAAAAAATGCAAAATCAGGAAAAATGTATCTAAACT ATTACTTTTACAAAGGCAAAAACAGCCTGTTTGCTATCT AATCAGTCCTGTTGATAAAACTCAGGCTGTACTGTTCTC TTCAGAGAGAGGCAAATGGGGGGAGTTATAAGATTTGC GCCCTACTGCTGTTAGTAGAATGAACAAGACAGAGTAA AAGCCACAAGTGGAATTGTGCTAGTATTTCCTCTATAAT TAACCCAACACTTAGGTAGCCTTTAAAGTGAAAGGAGC CCTCCAAATGATGGAGGCACACAGGAGGTAATTATGTG CTTTCAATAAATGCCTGAAAGCTCACAATTTCTTTGGGG AGATACAATCTCTGGAAAGGCTTGCATTGTCACTCCAAT TTCATTTATTTATTTTAACAAATTGTATGTTCTGTGGAGG AACTTTATTTCCCAAAAAGATGAACGTATAATAAACCA GTTCTTTATTTTAAAACTTATGGTGAGAGAAATTTCAAT TTTAGATAATTCCATTGTCTATAAAGAGTCTCTATTAAA TTAGCTTAAGTTCCAAAAGATAATGCCACATTATTATTG TAGGTAGCCATTATAATTCTCAAATTTCTATTGTTACATT CTGCAAGACAGTGGTAAGCAAACCACAACG |
| 18 | GSH3-RHA | TCATGGGCCAAAATGAATGGAATCAAAACCTGAGACAC ATTTCTGTTAATGGGGCTCTATTTGAGAAATGTGCATTA TTTTATCAGCTTCCTTATTTTAACCATTCTACCCTGTCAA TACTAATACCAAGTGTTCATTTTCTTTATTAGTTTAAATG GGAGAAAACAATGCAGAAAGCTTAATAACTGTTCTATT |
| | | TTTAAAGCCTTTATAGAAAACATAATATAAAAAGTGAT ATGAACTAGGCACAAGACAAAACTGACTTTAATATGTT ATATGTTAATCTGTTAAAATACAGTACATTTTACTGCAG AATGTTAGCAGGGTATATTTGTAGATAATAGATCATTGA CATTTTTATCCTATATCCTTTTTACCTTTTATTCCTTTTAA ACATTGATTACCTTTGTTATAAAAAGCAACAACAATAGT TTGTAAAAGATTAAGATATTTAAATATTTAAAAATTTTT CATAACTATATTAGTAAACAGTAGTACATAAGTCTTGAT AGGTACAAATGATGTCTCAAAAGACAGATGAGGTGGAA AATTACCACTCTAGCTCATGGCATGACCCCAGCCGTGCA GAGGAAAGATTTCTTCTCCTCCCCTATTCACCTCCGGGT CCCAGCTATCACTAAATATATCGGGGGTCGCGTTGCTGA TCAGCAGCCTTAAGCCTGAACATGGTGGGTCCTTTGCAT TAGTTCTCAATTCCCACCACCCTCCTCCCTTCCTGTGTCA CGAGGCTCACTCATTTCCTCAGTGCCCTTCCCAGTTACT GTCAAATCATATAAACTCATTTCTCTGCCTCCAGCCTTTT ATTTCATC |
| 19 | GSH3-LHA | GGCTTCCCTCTGTGATGGGTAGATAATTTTGCTTAATAT GTAAACAGTCTTTTCAACACTCTGTGCTTAAATAATCAT TGTTGGTTTGCTTTCTTCTAGTATTCCATATTAAGTAATG ACTTGGCTAATGAGAAGGATGCTTGTAAGTATATTAGC ATCAAGTTCAAATATAGAGAGATTAAATAACCTTCCCA AGGTCAACACACTGGTCATTGACAAATATGGACCAGAT CTCATCTCCAAAACCCCACACAATTATACCAGGTTAGCT CATTCTTTCTTCCTAGACATCTTATTTTCCAGGGAGTAGT GAAGGTCTACAAAACTTTCCTACTAAATAGGAAATATA TTTTGTTGTCAGATGTAAGCCCAGAAAAGCAATGGCTA AACTGCTATGTATCAAAAACTTTGCAAACTTATAACAGT TTAAAATTTACTTCTAGCTATGTTTAGAGCCCTAAGGAG ATAATGGCAAGTCATTTTTTCTTCTTTATACATATGTCAT CATTTATGGACAGAAAATAGTTCTCAATTGTAAAAAAA TAGCTCTCTATAGGATTTGCTGAGTTTTTTATGGCTAGA GTTATTAAGTAAAATAAATGACATCTGAGAGCACTCTG CTTTGTCTTTGGCTTCAAATAATGTATTTTACTATATTTT TTCATTCTTCGCTGCCATAAGTGGCAGAAAGAAAACCG CTTTTCAAATGGCTAGTGCCTAAAAAGCAAATACTTTGT GGATCTTGGGGCCAGTGTAAACTCAGAATGCAAGATAT CTTCCTCAGCTTCAGTACTTGCACAATGCCTGTCTTTATT AAACATGAGTGATTTGCAAATATGATGTTTCTGAGACA GCTAATA |
| 20 | AAVS1-RHA | ACTAGGGACAGGATTGGTGACAGAAAAGCCCCATCCTT AGGCCTCCTCCTTCCTAGTCTCCTGATATTGGGTCTAAC CCCCACCTCCTGTTAGGCAGATTCCTTATCTGGTGACAC ACCCCCATTTCCTGGAGCCATCTCTCTCCTTGCCAGAAC CTCTAAGGTTTGCTTACGATGGAGCCAGAGAGGATCCT GGGAGGGAGAGCTTGGCAGGGGGTGGGAGGGAAGGGG GGGATGCGTGACCTGCCCGGTTCTCAGTGGCCACCCTGC GCTACCCTCTCCCAGAACCTGAGCTGCTCTGACGCGGCT GTCTGGTGCGTTTCACTGATCCTGGTGCTGCAGCTTCCT TACACTTCCCAAGAGGAGAAGCAGTTTGGAAAAACAAA ATCAGAATAAGTTGGTCCTGAGTTCTAACTTTGGCTCTT CACCTTTCTAGTCCCCAATTTATATTGTTCCTCCGTGCGT CAGTTTTACCTGTGAGATAAGGCCAGTAGCCAGCCCCGT CCTGGCAGGGCTGTGGTGAGGAGGGGGGTGTCCGTGTG |
| | | GAAAACTCCCTTTGTGAGAATGGTGCGTCCTAGGTGTTC ACCAGGTCGTGGCCGCCTCTACTCCCTTTCTCTTTCTCCA TCCTTCTTTCCTTAAAGAGTCCCCAGTGCTATCTGGGAC ATATTCCTCCGCCCAGAGCAGGGTCCCGCTTCCCTAAGG CCCTGCTCTGGGCTTCTGGGTTTGAGTCCTTGGCAAGCC CAGGAGAGGCGCTCAGGCTTCCCTGTCCCCCTTCCTCGT CCACCATCTCATGCCCCTGGCTCTCCTGCCCCTTCCCTAC AGGGGTTCCTGGCTCTGCTCT |
| 21 | AAVS1-LHA | TGCTTTCTCTGACCAGCATTCTCTCCCCTGGGCCTGTGCC GCTTTCTGTCTGCAGCTTGTGGCCTGGGTCACCTCTACG GCTGGCCCAGATCCTTCCCTGCCGCCTCCTTCAGGTTCC GTCTTCCTCCACTCCCTCTTCCCCTTGCTCTCTGCTGTGT TGCTGCCCAAGGATGCTCTTTCCGGAGCACTTCCTTCTC GGCGCTGCACCACGTGATGTCCTCTGAGCGGATCCTCCC CGTGTCTGGGTCCTCTCCGGGCATCTCTCCTCCCTCACC CAACCCCATGCCGTCTTCACTCGCTGGGTTCCCTTTTCCT TCTCCTTCTGGGGCCTGTGCCATCTCTCGTTTCTTAGGAT GGCCTTCTCCGACGGATGTCTCCCTTGCGTCCCGCCTCC CCTTCTTGTAGGCCTGCATCATCACCGTTTTTCTGGACA ACCCCAAAGTACCCCGTCTCCCTGGCTTTAGCCACCTCT CCATCCTCTTGCTTTCTTTGCCTGGACACCCCGTTCTCCT GTGGATTCGGGTCACCTCTCACTCCTTTCATTTGGGCAG CTCCCCTACCCCCCTTACCTCTCTAGTCTGTGCTAGCTCT TCCAGCCCCCTGTCATGGCATCTTCCAGGGGTCCGAGAG CTCAGCTAGTCTTCTTCCTCCAACCCGGGCCCCTATGTC CACTTCAGGACAGCATGTTTGCTGCCTCCAGGGATCCTG TGTCCCCGAGCTGGGACCACCTTATATTCCCAGGGCCGG TTAATGTGGCTCTGGTTCTGGGTACTTTTATCTGTCCCCT CCACCCCACAGTGGGGC |
| 22 | GSH1 donor vector | TGCAGCTCTGGCCCGTGTCTCAAAATCTCTGATGTTACA TTGCACAAGATAAAAATATATCATCATGAACAATAAAA CTGTCTGCTTACATAAACAGTAATACAAGGGGTGTTATG AGCCATATTCAACGGGAAACGTCGAGGCCGCGATTAAA TTCCAACATGGATGCTGATTTATATGGGTATAAATGGGC TCGCGATAATGTCGGGCAATCAGGTGCGACAATCTATC GCTTGTATGGGAAGCCCGATGCGCCAGAGTTGTTTCTGA AACATGGCAAAGGTAGCGTTGCCAATGATGTTACAGAT GAGATGGTCAGACTAAACTGGCTGACGGAATTTATGCC TCTTCCGACCATCAAGCATTTTATCCGTACTCCTGATGA TGCATGGTTACTCACCACTGCGATCCCCGGAAAAACAG CATTCCAGGTATTAGAAGAATATCCTGATTCAGGTGAA AATATTGTTGATGCGCTGGCAGTGTTCCTGCGCCGGTTG CATTCGATTCCTGTTTGTAATTGTCCTTTTAACAGCGATC GCGTATTTCGTCTCGCTCAGGCGCAATCACGAATGAATA ACGGTTTGGTTGATGCGAGTGATTTTGATGACGAGCGTA ATGGCTGGCCTGTTGAACAAGTCTGGAAAGAAATGCAT AAACTTTTGCCATTCTCACCGGATTCAGTCGTCACTCAT GGTGATTTCTCACTTGATAACCTTATTTTTGACGAGGGG AAATTAATAGGTTGTATTGATGTTGGACGAGTCGGAATC GCAGACCGATACCAGGATCTTGCCATCCTATGGAACTG CCTCGGTGAGTTTTCTCCTTCATTACAGAAACGGCTTTTT CAAAAATATGGTATTGATAATCCTGATATGAATAAATTG CAGTTTCATTTGATGCTCGATGAGTTTTTCTAATCAGAA TTGGTTAATTGGTTGTAACATTATTCAGATTGGGCTTGA |
| | | TTTAAAACTTCATTTTTAATTTAAAAGGATCTAGGTGAA GATCCTTTTTGATAATCTCATGACCAAAATCCCTTAACG TGAGTTTTCGTTCCACTGAGCGTCAGACCCCGTAGAAAA GATCAAAGGATCTTCTTGAGATCCTTTTTTTCTGCGCGT AATCTGCTGCTTGCAAACAAAAAAACCACCGCTACCAG CGGTGGTTTGTTTGCCGGATCAAGAGCTACCAACTCTTT TTCCGAAGGTAACTGGCTTCAGCAGAGCGCAGATACCA AATACTGTTCTTCTAGTGTAGCCGTAGTTAGGCCACCAC TTCAAGAACTCTGTAGCACCGCCTACATACCTCGCTCTG CTAATCCTGTTACCAGTGGCTGCTGCCAGTGGCGATAAG TCGTGTCTTACCGGGTTGGACTCAAGACGATAGTTACCG GATAAGGCGCAGCGGTCGGGCTGAACGGGGGGTTCGTG CACACAGCCCAGCTTGGAGCGAACGACCTACACCGAAC TGAGATACCTACAGCGTGAGCTATGAGAAAGCGCCACG CTTCCCGAAGGGAGAAAGGCGGACAGGTATCCGGTAAG CGGCAGGGTCGGAACAGGAGAGCGCACGAGGGAGCTTC CAGGGGGAAACGCCTGGTATCTTTATAGTCCTGTCGGGT TTCGCCACCTCTGACTTGAGCGTCGATTTTTGTGATGCT CGTCAGGGGGGCGGAGCCTATGGAAAAACGCCAGCAAC GCGGCCTTTTTACGGTTCCTGGCCTTTTGCTGGCCTTTTG CTCACATGTTCTTTCCTGCGTTATCCCCTGATTCTGTGGA TAACCGTATTACCGCCTTTGAGTGAGCTGATACCGCTCG CCGCAGCCGAACGACCGAGCGCAGCGAGTCAGTGAGCG AGGAAGCGGAAGAGCGCCCAATACGCAAACCGCCTCTC CCCGCGCGTTGGCCGATTCATTAATGCAGCTGGCACGAC AGGTTTCCCGACTGGAAAGCGGGCAGTGAGCGCAACGC AATTAATGTGAGTTAGCTCACTCATTAGGCACCCCAGGC TTTACACTTTATGCTTCCGGCTCGTATGTTGTGTGGAATT GTGAGCGGATAACAATTTCACACAGGAAACAGCTATGA CCATGATTACGCCGCGGCCGCGCAAATAGCAAGTCTTCT CTGGTTCTTTGAGCTCTGTTTTTGATGACTTCACCCTGGA GGTAACAGCTTTCCTAAGGCCTGACATGCAGGCCTGCTG CATTGCAAACTCCCTCTGCTCATTTTTGGCAGGTTGTGC TGATTTATGGGGCTATTTGATTTATTTAATGTACCATGC CTTTGATTGCATATACTCTATAGCAGTCACTCAAAATGC AAACAAACACATCATATGTTCTTTAATTACTCCCATCCC CCTTATTATCAATCACTGCCAATATCAGCAGAATGGCTG TGATTCATGCTATAAAATTAAGGTCAGCAAACTTGGGTT TTGCAGAAGTGAATAGCAGCAATTCTGTTTATATTCTTT AATGTTTCAATTCTAAAACTCATTATCAGAAAAATGGTA TTTCAGATCAGTCTTGTTAGAAGAATGGGATGGGAAAA AAGATATAACAGTTGGGCAAAGAGAAGGAAAGCAAAT GTAGCACTAGGGAAAAAGTTGTAGAAGAAAGTAAAAA AAAAAATAAAGATGAGAAGATAGTAGCTCTTAAGGATA TTCATTCTGACTTATGAAAGACTGTAATGTTTAGCACAC ACAGACACATACACAAACACATGAACTTGTAAAATTCT CAAAGGTCCAGTTTTTATTCAAAATTCCTATACTTCAAC TTCTTGAATCTCACTCCTTTTTTTTTCAAGTTCCTCATTTC TCTCCCTTAAAAAATGGATGCTAACCATTTTAAGCTTAG TAATTTTGTACATGCTTTTCTGGAAATACTACAAATATG ATCAATGTCTGTGATCTCTTCAGTGCTCTACTCTGCGGC CGCATAACTTCGTATAGCATACATTATACGAAGTTATCT GACCTCTTCTCTTCCTCCCACAGGGCCTCGAGAGATCTG GCAGCGGAGAGGGCAGAGGAAGTCTTCTAACATGCGGT |
| | | GACGTGGAGGAGAATCCCGGCCCTAGGATGACCGAGTA CAAGCCCACGGTGCGCCTCGCCACCCGCGACGACGTCC CCAGGGCCGTACGCACCCTCGCCGCCGCGTTCGCCGACT ACCCCGCCACGCGCCACACCGTCGATCCGGACCGCCAC ATCGAGCGGGTCACCGAGCTGCAAGAACTCTTCCTCAC GCGCGTCGGGCTCGACATCGGCAAGGTGTGGGTCGCGG ACGACGGCGCCGCGGTGGCGGTCTGGACCACGCCGGAG AGCGTCGAAGCGGGGGCGGTGTTCGCCGAGATCGGCCC GCGCATGGCCGAGTTGAGCGGTTCCCGGCTGGCCGCGC AGCAACAGATGGAAGGCCTCCTGGCGCCGCACCGGCCC AAGGAGCCCGCGTGGTTCCTGGCCACCGTCGGCGTCTC GCCCGACCACCAGGGCAAGGGTCTGGGCAGCGCCGTCG TGCTCCCCGGAGTGGAGGCGGCCGAGCGCGCCGGGGTG CCCGCCTTCCTGGAGACCTCCGCGCCCCGCAACCTCCCC TTCTACGAGCGGCTCGGCTTCACCGTCACCGCCGACGTC GAGGTGCCCGAAGGACCGCGCACCTGGTGCATGACCCG CAAGCCCGGTGCCTGACTGTGCCTTCTAGTTGCCAGCCA TCTGTTGTTTGCCCCTCCCCCGTGCCTTCCTTGACCCTGG AAGGTGCCACTCCCACTGTCCTTTCCTAATAAAATGAGG AAATTGCATCGCATTGTCTGAGTAGGTGTCATTCTATTC TGGGGGGTGGGGTGGGGCAGGACAGCAAGGGGGAGGA TTGGGAAGACAATAGCAGGCATGCTGGGGATGCGGTGG GCTCTATGGATAACTTCGTATAGCATACATTATACGAAG TTATGAGCTCACGGGGACAGCCCCCCCCCAAAGCCCCC AGGGATGTAATTACGTCCCTCCCCCGCTAGGGGGCAGC AGCGAGCCGCCCGGGGCTCCGCTCCGGTCCGGCGCTCC CCCCGCATCCCCGAGCCGGCAGCGTGCGGGGACAGCCC GGGCACGGGGAAGGTGGCACGGGATCGCTTTCCTCTGA ACGCTTCTCGCTGCTCTTTGAGCCTGCAGACACCTGGGG GGATACGGGGAAAAAGCTTTAGGCTGAAAGAGAGATTT AGAATGACAGAATCATAGAACGGCCTGGGTTGCAAAGG AGCACAGTGCTCATCCAGATCCAACCCCCTGCTATGTGC AGGGTCATCAACCAGCAGCCCAGGCTGCCCAGAGCCAC ATCCAGCCTGGCCTTGAATGCCTGCAGGGATGGGGCAT CCACAGCCTCCTTGGGCAACCTGTTCAGTGCGTCACCAC CCTCTGGGGGAAAAACTGCCTCCTCATATCCAACCCAA ACCTCCCCTGTCTCAGTGTAAAGCCATTCCCCCTTGTCC TATCAAGGGGGAGTTTGCTGTGACATTGTTGGTCTGGGG TGACACATGTTTGCCAATTCAGTGCATCACGGAGAGGC AGATCTTGGGGATAAGGAAGTGCAGGACAGCATGGACG TGGGACATGCAGGTGTTGAGGGCTCTGGGACACTCTCC AAGTCACAGCGTTCAGAACAGCCTTAAGGATAAGAAGA TAGGATAGAAGGACAAAGAGCAAGTTAAAACCCAGCAT GGAGAGGAGCACAAAAAGGCCACAGACACTGCTGGTCC CTGTGTCTGAGCCTGCATGTTTGATGGTGTCTGGATGCA AGCAGAAGGGGTGGAAGAGCTTGCCTGGAGAGATACAG CTGGGTCAGTAGGACTGGGACAGGCAGCTGGAGAATTG CCATGTAGATGTTCATACAATCGTCAAATCATGAAGGCT GGAAAAGCCCTCCAAGATCCCCAAGACCAACCCCAACC CACCCACCGTGCCCACTGGCCATGTCCCTCAGTGCCACA TCCCCACAGTTCTTCATCACCTCCAGGGACGGTGACCCC CCCACCTCCGTGGGCAGCTGTGCCACTGCAGCACCGCTC TTTGGAGAAGGTAAATCTTGCTAAATCCAGCCCGACCCT CCCCTGGCACAACGTAAGGCCATTATCTCTCATCCAACT |
| | | CCAGGACGGAGTCAGTGAGAATATTGGCGCGCCGAATT CTCGCGACGATCGGTCGACATAACTTCGTATAGGATACT TTATACGAAGTTATCAATTCAGGCTAGTTATTAATAGTA ATCAATTACGGGGTCATTAGTTCATAGCCCATATATGGA GTTCCGCGTTACATAACTTACGGTAAATGGCCCGCCTGG CTGACCGCCCAACGACCCCCGCCCATTGACGTCAATAAT GACGTATGTTCCCATAGTAACGCCAATAGGGACTTTCCA TTGACGTCAATGGGTGGAGTATTTACGGTAAACTGCCCA CTTGGCAGTACATCAAGTGTATCATATGCCAAGTACGCC CCCTATTGACGTCAATGACGGTAAATGGCCCGCCTGGC ATTATGCCCAGTACATGACCTTATGGGACTTTCCTACTT GGCAGTACATCTACGTATTAGTCATCGCTATTACCATGG TCGAGGTGAGCCCCACGTTCTGCTTCACTCTCCCCATCT CCCCCCCCTCCCCACCCCCAATTTTGTATTTATTTATTTT TTAATTATTTTGTGCAGCGATGGGGGCGGGGGGGGGGG GGGGGCGCGCGCCAGGCGGGGCGGGGCGGGGCGAGGG GCGGGGCGGGGCGAGGCGGAGAGGTGCGGCGGCAGCC AATCAGAGCGGCGCGCTCCGAAAGTTTCCTTTTATGGCG AGGCGGCGGCGGCGGCGGCCCTATAAAAAGCGAAGCGC GCGGCGGGCGGGGAGTCGCTGCGACGCTGCCTTCGCCC CGTGCCCCGCTCCGCCGCCGCCTCGCGCCGCCCGCCCCG GCTCTGACTGACCGCGTTACTCCCACAGGTGAGCGGGC GGGACGGCCCTTCTCCTCCGGGCTGTAATTAGCGCTTGG TTTAATGACGGCTTGTTTCTTTTCTGTGGCTGCGTGAAA GCCTTGAGGGGCTCCGGGAGGGCCCTTTGTGCGGGGGG AGCGGCTCGGGGGGTGCGTGCGTGTGTGTGTGCGTGGG GAGCGCCGCGTGCGGCTCCGCGCTGCCCGGCGGCTGTG AGCGCTGCGGGCGCGGCGCGGGGCTTTGTGCGCTCCGC AGTGTGCGCGAGGGGAGCGCGGCCGGGGGCGGTGCCCC GCGGTGCGGGGGGGGCTGCGAGGGGAACAAAGGCTGC GTGCGGGGTGTGTGCGTGGGGGGGTGAGCAGGGGGTGT GGGCGCGTCGGTCGGGCTGCAACCCCCCCTGCACCCCC CTCCCCGAGTTGCTGAGCACGGCCCGGCTTCGGGTGCG GGGCTCCGTACGGGGCGTGGCGCGGGGCTCGCCGTGCC GGGCGGGGGGTGGCGGCAGGTGGGGGTGCCGGGCGGG GCGGGGCCGCCTCGGGCCGGGGAGGGCTCGGGGGAGG GGCGCGGCGGCCCCCGGAGCGCCGGCGGCTGTCGAGGC GCGGCGAGCCGCAGCCATTGCCTTTTATGGTAATCGTGC GAGAGGGCGCAGGGACTTCCTTTGTCCCAAATCTGTGC GGAGCCGAAATCTGGGAGGCGCCGCCGCACCCCCTCTA GCGGGCGCGGGGCGAAGCGGTGCGGCGCCGGCAGGAA GGAAATGGGCGGGGAGGGCCTTCGTGCGTCGCCGCGCC GCCGTCCCCTTCTCCCTCTCCAGCCTCGGGGCTGTCCGC GGGGGGACGGCTGCCTTCGGGGGGGACGGGGCAGGGC GGGGTTCGGCTTCTGGCGTGTGACCGGCGGCTCTAGAG CCTCTGCTAACCATGTTCATGCCTTCTTCTTTTTCCTACA GCTCCTGGGCAACGTGCTGGTTATTGTGCTGTCTCATCA TTTTGGCAAAGAATTGTGTACAACCATGGAGAGCGACG AGAGCGGCCTGCCCGCCATGGAGATCGAGTGCCGCATC ACCGGCACCCTGAACGGCGTGGAGTTCGAGCTGGTGGG CGGCGGAGAGGGCACCCCCAAGCAGGGCCGCATGACCA ACAAGATGAAGAGCACCAAAGGCGCCCTGACCTTCAGC CCCTACCTGCTGAGCCACGTGATGGGCTACGGCTTCTAC CACTTCGGCACCTACCCCAGCGGCTACGAGAACCCCTTC |
| | | CTGCACGCCATCAACAACGGCGGCTACACCAACACCCG CATCGAGAAGTACGAGGACGGCGGCGTGCTGCACGTGA GCTTCAGCTACCGCTACGAGGCCGGCCGCGTGATCGGC GACTTCAAGGTGGTGGGCACCGGCTTCCCCGAGGACAG CGTGATCTTCACCGACAAGATCATCCGCAGCAACGCCA CCGTGGAGCACCTGCACCCCATGGGCGATAACGTGCTG GTGGGCAGCTTCGCCCGCACCTTCAGCCTGCGCGACGG CGGCTACTACAGCTTCGTGGTGGACAGCCACATGCACTT CAAGAGCGCCATCCACCCCAGCATCCTGCAGAACGGGG GCCCCATGTTCGCCTTCCGCCGCGTGGAGGAGCTGCACA GCAACACCGAGCTGGGCATCGTGGAGTACCAGCACGCC TTCAAGACCCCCATCGCCTTCGCCAGATCCCGCGCTCAG TCGTCCAATTCTGCCGTGGACGGCACCGCCGGACCCGG CTCCACCGGATCTCGCTAAACGCGTGAATTCACTCCTCA GGTGCAGGCTGCCTATCAGAAGGTGGTGGCTGGTGTGG CCAATGCCCTGGCTCACAAATACCACTGAGATCTTTTTC CCTCTGCCAAAAATTATGGGGACATCATGAAGCCCCTTG AGCATCTGACTTCTGGCTAATAAAGGAAATTTATTTTCA TTGCAATAGTGTGTTGGAATTTTTTGTGTCTCTCACTCGG AAGGACATATGGGAGGGCAAATCATTTAAAACATCAGA ATGAGTATTTGGTTTAGAGTTTGGCAACATATGCCCATA TGCTGGCTGCCATGAACAAAGGTTGGCTATAAAGAGGT CATCAGTATATGAAACAGCCCCCTGCTGTCCATTCCTTA TTCCATAGAAAAGCCTTGACTTGAGGTTAGATTTTTTTT ATATTTTGTTTTGTGTTATTTTTTTCTTTAACATCCCTAA AATTTTCCTTACATGTTTTACTAGCCAGATTTTTCCTCCT CTCCTGACTACTCCCAGTCATAGCTGTCCCTCTTCTCTTA TGGAGATCACCGGTATAACTTCGTATAATGTATACTATA CGAAGTTATGGTACCTTAATTAAGTTAACATGCATAGTA CTGAGCTCACGGGGACAGCCCCCCCCCAAAGCCCCCAG GGATGTAATTACGTCCCTCCCCCGCTAGGGGGCAGCAG CGAGCCGCCCGGGGCTCCGCTCCGGTCCGGCGCTCCCC CCGCATCCCCGAGCCGGCAGCGTGCGGGGACAGCCCGGG CACGGGGAAGGTGGCACGGGATCGCTTTCCTCTGAACG CTTCTCGCTGCTCTTTGAGCCTGCAGACACCTGGGGGGA TACGGGGAAAAAGCTTTAGGCTGAAAGAGAGATTTAGA ATGACAGAATCATAGAACGGCCTGGGTTGCAAAGGAGC ACAGTGCTCATCCAGATCCAACCCCCTGCTATGTGCAGG GTCATCAACCAGCAGCCCAGGCTGCCCAGAGCCACATC CAGCCTGGCCTTGAATGCCTGCAGGGATGGGGCATCCA CAGCCTCCTTGGGCAACCTGTTCAGTGCGTCACCACCCT CTGGGGGAAAAACTGCCTCCTCATATCCAACCCAAACC TCCCCTGTCTCAGTGTAAAGCCATTCCCCCTTGTCCTATC AAGGGGGAGTTTGCTGTGACATTGTTGGTCTGGGGTGA CACATGTTTGCCAATTCAGTGCATCACGGAGAGGCAGA TCTTGGGGATAAGGAAGTGCAGGACAGCATGGACGTGG GACATGCAGGTGTTGAGGGCTCTGGGACACTCTCCAAG TCACAGCGTTCAGAACAGCCTTAAGGATAAGAAGATAG GATAGAAGGACAAAGAGCAAGTTAAAACCCAGCATGG AGAGGAGCACAAAAAGGCCACAGACACTGCTGGTCCCT GTGTCTGAGCCTGCATGTTTGATGGTGTCTGGATGCAAG CAGAAGGGGTGGAAGAGCTTGCCTGGAGAGATACAGCT GGGTCAGTAGGACTGGGACAGGCAGCTGGAGAATTGCC ATGTAGATGTTCATACAATCGTCAAATCATGAAGGCTG |
| | | GAAAAGCCCTCCAAGATCCCCAAGACCAACCCCAACCC ACCCACCGTGCCCACTGGCCATGTCCCTCAGTGCCACAT CCCCACAGTTCTTCATCACCTCCAGGGACGGTGACCCCC CCACCTCCGTGGGCAGCTGTGCCACTGCAGCACCGCTCT TTGGAGAAGGTAAATCTTGCTAAATCCAGCCCGACCCTC CCCTGGCACAACGTAAGGCCATTATCTCTCATCCAACTC CAGGACGGAGTCAGTGAGAATATTGTTTAAACTGTGGG AAGCTTCTTTCCAACTTGGTTCCTGTCCATACCCACGTA AAATATTAAAGAAAGAAGGATAGAAATATAGATTAAAA GAAGACCTGAAGTCGAAGGAAGGGACTAATTGTTGAGA GATTTGAAATGCAACCAATTTTCCATCAGATGTTTTATC GGTACTCTGTAGTTCTATTCCGTCCCAGTTTTCCATAAAT TTATAGATAAAAAGAATATTTTATATTGCATTTTGTATG CATATATGCATACATACATATCACTTAAATATATATATA TATAAAATTGCATGTTATGCACACTTTAAAATCTTTAAA ATATTGAATGCTTAATTACTTAGGACAAAATAGCTTACA TAGGAAACAATTATTTCGGACAGAATAATTTAGGAGAC AATAGCATTTTTGTGCTGTTGCAGAGATGATAAAGAACT TCTGGTTTTATATGTAACTCTAAAACTGCAGGATAAATA GGTACGTAATACAAATGGCAGTTGCCTCACTGCAATTTT CTGAGCTGTGTTGCTAGTCTGACACTCCTGAAAATTAAT ATTTCAGACATCTCTGTAATTGTCCAGAGGGCCTGGAGT GCCTTCAAATTATATTCCCCCTCTATTTTAATCAACCATG TTAGTAATTTACTTTTCATTAGTTTATTGATAATTCTATA GGGAAAGTTTGCTAATCACTGTGTAAAATATAATTTTCA AACAGCCAAAATAACTGCCATAAAATATGGAAACTGTC TTAGTTTGTTTTCTCTTGCTATGAAAGAATACCACAGAC TGGGTAATGTATAGAGAAGAGGTTTAAACACTGGCCGT CGTTTTACAACGTCGTGACTGGGAAAACCCTGGCGTTAC CCAACTTAATCGCCTTGCAGCACATCCCCCTTTCGCCAG CTGGCGTAATAGCGAAGAGGCCCGCACCGATCGCCCTT CCCAACAGTTGCGCAGCCTGAATGGCGAATGGCGCCTG ATGCGGTATTTTCTCCTTACGCATCTGTGCGGTATTTCAC ACCGCATATGGTGCACTCTCAGTACAATCTGCTCTGATG CCGCATAGTTAAGCCAGCCCCGACACCCGCCAACACCC GCTGACGCGCCCTGACGGGCTTGTCTGCTCCCGGCATCC GCTTACAGACAAGCTGTGACCGTCTCCGGGAGCTGCAT GTGTCAGAGGTTTTCACCGTCATCACCGAAACGCGCGA |
| 23 | GSH2 donor vector | TGCAGCTCTGGCCCGTGTCTCAAAATCTCTGATGTTACA TTGCACAAGATAAAAATATATCATCATGAACAATAAAA CTGTCTGCTTACATAAACAGTAATACAAGGGGTGTTATG AGCCATATTCAACGGGAAACGTCGAGGCCGCGATTAAA TTCCAACATGGATGCTGATTTATATGGGTATAAATGGGC TCGCGATAATGTCGGGCAATCAGGTGCGACAATCTATC GCTTGTATGGGAAGCCCGATGCGCCAGAGTTGTTTCTGA AACATGGCAAAGGTAGCGTTGCCAATGATGTTACAGAT GAGATGGTCAGACTAAACTGGCTGACGGAATTTATGCC TCTTCCGACCATCAAGCATTTTATCCGTACTCCTGATGA TGCATGGTTACTCACCACTGCGATCCCCGGAAAAACAG CATTCCAGGTATTAGAAGAATATCCTGATTCAGGTGAA AATATTGTTGATGCGCTGGCAGTGTTCCTGCGCCGGTTG CATTCGATTCCTGTTTGTAATTGTCCTTTTAACAGCGATC GCGTATTTCGTCTCGCTCAGGCGCAATCACGAATGAATA ACGGTTTGGTTGATGCGAGTGATTTTGATGACGAGCGTA |
| | | ATGGCTGGCCTGTTGAACAAGTCTGGAAAGAAATGCAT AAACTTTTGCCATTCTCACCGGATTCAGTCGTCACTCAT GGTGATTTCTCACTTGATAACCTTATTTTTGACGAGGGG AAATTAATAGGTTGTATTGATGTTGGACGAGTCGGAATC GCAGACCGATACCAGGATCTTGCCATCCTATGGAACTG CCTCGGTGAGTTTTCTCCTTCATTACAGAAACGGCTTTTT CAAAAATATGGTATTGATAATCCTGATATGAATAAATTG CAGTTTCATTTGATGCTCGATGAGTTTTTCTAATCAGAA TTGGTTAATTGGTTGTAACATTATTCAGATTGGGCTTGA TTTAAAACTTCATTTTTAATTTAAAAGGATCTAGGTGAA GATCCTTTTTGATAATCTCATGACCAAAATCCCTTAACG TGAGTTTTCGTTCCACTGAGCGTCAGACCCCGTAGAAAA GATCAAAGGATCTTCTTGAGATCCTTTTTTTCTGCGCGT AATCTGCTGCTTGCAAACAAAAAAACCACCGCTACCAG CGGTGGTTTGTTTGCCGGATCAAGAGCTACCAACTCTTT TTCCGAAGGTAACTGGCTTCAGCAGAGCGCAGATACCA AATACTGTTCTTCTAGTGTAGCCGTAGTTAGGCCACCAC TTCAAGAACTCTGTAGCACCGCCTACATACCTCGCTCTG CTAATCCTGTTACCAGTGGCTGCTGCCAGTGGCGATAAG TCGTGTCTTACCGGGTTGGACTCAAGACGATAGTTACCG GATAAGGCGCAGCGGTCGGGCTGAACGGGGGGTTCGTG CACACAGCCCAGCTTGGAGCGAACGACCTACACCGAAC TGAGATACCTACAGCGTGAGCTATGAGAAAGCGCCACG CTTCCCGAAGGGAGAAAGGCGGACAGGTATCCGGTAAG CGGCAGGGTCGGAACAGGAGAGCGCACGAGGGAGCTTC CAGGGGGAAACGCCTGGTATCTTTATAGTCCTGTCGGGT TTCGCCACCTCTGACTTGAGCGTCGATTTTTGTGATGCT CGTCAGGGGGGCGGAGCCTATGGAAAAACGCCAGCAAC GCGGCCTTTTTACGGTTCCTGGCCTTTTGCTGGCCTTTTG CTCACATGTTCTTTCCTGCGTTATCCCCTGATTCTGTGGA TAACCGTATTACCGCCTTTGAGTGAGCTGATACCGCTCG CCGCAGCCGAACGACCGAGCGCAGCGAGTCAGTGAGCG AGGAAGCGGAAGAGCGCCCAATACGCAAACCGCCTCTC CCCGCGCGTTGGCCGATTCATTAATGCAGCTGGCACGAC AGGTTTCCCGACTGGAAAGCGGGCAGTGAGCGCAACGC AATTAATGTGAGTTAGCTCACTCATTAGGCACCCCAGGC TTTACACTTTATGCTTCCGGCTCGTATGTTGTGTGGAATT GTGAGCGGATAACAATTTCACACAGGAAACAGCTATGA CCATGATTACGCCGCGGCCGCCAGACACATGTTCCCCA GAAGAGGATTTGCCATTCTGACAAATCTCCATCCAAGG AATGGGAAATTCATTATCCCTTCCACTAGAATTAAACTG TGAAATTATTTATGTAGGAAGAAGTAGTAGTAGAAAAG TTATGTTTGTCTTCTTCACTATAAGAAAAAAATGCAAAA TCAGGAAAAATGTATCTAAACTATTACTTTTACAAAGGC AAAAACAGCCTGTTTGCTATCTAATCAGTCCTGTTGATA AAACTCAGGCTGTACTGTTCTCTTCAGAGAGAGGCAAA TGGGGGGAGTTATAAGATTTGCGCCCTACTGCTGTTAGT AGAATGAACAAGACAGAGTAAAAGCCACAAGTGGAATT GTGCTAGTATTTCCTCTATAATTAACCCAACACTTAGGT AGCCTTTAAAGTGAAAGGAGCCCTCCAAATGATGGAGG CACACAGGAGGTAATTATGTGCTTTCAATAAATGCCTGA AAGCTCACAATTTCTTTGGGGAGATACAATCTCTGGAAA GGCTTGCATTGTCACTCCAATTTCATTTATTTATTTTAAC AAATTGTATGTTCTGTGGAGGAACTTTATTTCCCAAAAA |
| | | GATGAACGTATAATAAACCAGTTCTTTATTTTAAAACTT ATGGTGAGAGAAATTTCAATTTTAGATAATTCCATTGTC TATAAAGAGTCTCTATTAAATTAGCTTAAGTTCCAAAAG ATAATGCCACATTATTATTGTAGGTAGCCATTATAATTC TCAAATTTCTATTGTTACATTCTGCAAGACAGTGGTAAG CAAACCACAACGGCGGCCGCATAACTTCGTATAGCATA CATTATACGAAGTTATCTGACCTCTTCTCTTCCTCCCACA GGGCCTCGAGAGATCTGGCAGCGGAGAGGGCAGAGGA AGTCTTCTAACATGCGGTGACGTGGAGGAGAATCCCGG CCCTAGGATGACCGAGTACAAGCCCACGGTGCGCCTCG CCACCCGCGACGACGTCCCCAGGGCCGTACGCACCCTC GCCGCCGCGTTCGCCGACTACCCCGCCACGCGCCACAC CGTCGATCCGGACCGCCACATCGAGCGGGTCACCGAGC TGCAAGAACTCTTCCTCACGCGCGTCGGGCTCGACATCG GCAAGGTGTGGGTCGCGGACGACGGCGCCGCGGTGGCG GTCTGGACCACGCCGGAGAGCGTCGAAGCGGGGGCGGT GTTCGCCGAGATCGGCCCGCGCATGGCCGAGTTGAGCG GTTCCCGGCTGGCCGCGCAGCAACAGATGGAAGGCCTC CTGGCGCCGCACCGGCCCAAGGAGCCCGCGTGGTTCCT GGCCACCGTCGGCGTCTCGCCCGACCACCAGGGCAAGG GTCTGGGCAGCGCCGTCGTGCTCCCCGGAGTGGAGGCG GCCGAGCGCGCCGGGGTGCCCGCCTTCCTGGAGACCTC CGCGCCCCGCAACCTCCCCTTCTACGAGCGGCTCGGCTT CACCGTCACCGCCGACGTCGAGGTGCCCGAAGGACCGC GCACCTGGTGCATGACCCGCAAGCCCGGTGCCTGACTG TGCCTTCTAGTTGCCAGCCATCTGTTGTTTGCCCCTCCCC CGTGCCTTCCTTGACCCTGGAAGGTGCCACTCCCACTGT CCTTTCCTAATAAAATGAGGAAATTGCATCGCATTGTCT GAGTAGGTGTCATTCTATTCTGGGGGGTGGGGTGGGGC AGGACAGCAAGGGGGAGGATTGGGAAGACAATAGCAG GCATGCTGGGGATGCGGTGGGCTCTATGGATAACTTCGT ATAGCATACATTATACGAAGTTATGAGCTCACGGGGAC AGCCCCCCCCCAAAGCCCCCAGGGATGTAATTACGTCC CTCCCCCGCTAGGGGGCAGCAGCGAGCCGCCCGGGGCT CCGCTCCGGTCCGGCGCTCCCCCCGCATCCCCGAGCCGG CAGCGTGCGGGGACAGCCCGGGCACGGGGAAGGTGGC ACGGGATCGCTTTCCTCTGAACGCTTCTCGCTGCTCTTT GAGCCTGCAGACACCTGGGGGGATACGGGGAAAAAGCT TTAGGCTGAAAGAGAGATTTAGAATGACAGAATCATAG AACGGCCTGGGTTGCAAAGGAGCACAGTGCTCATCCAG ATCCAACCCCCTGCTATGTGCAGGGTCATCAACCAGCA GCCCAGGCTGCCCAGAGCCACATCCAGCCTGGCCTTGA ATGCCTGCAGGGATGGGGCATCCACAGCCTCCTTGGGC AACCTGTTCAGTGCGTCACCACCCTCTGGGGGAAAAAC TGCCTCCTCATATCCAACCCAAACCTCCCCTGTCTCAGT GTAAAGCCATTCCCCCTTGTCCTATCAAGGGGGAGTTTG CTGTGACATTGTTGGTCTGGGGTGACACATGTTTGCCAA TTCAGTGCATCACGGAGAGGCAGATCTTGGGGATAAGG AAGTGCAGGACAGCATGGACGTGGGACATGCAGGTGTT GAGGGCTCTGGGACACTCTCCAAGTCACAGCGTTCAGA ACAGCCTTAAGGATAAGAAGATAGGATAGAAGGACAA AGAGCAAGTTAAAACCCAGCATGGAGAGGAGCACAAA AAGGCCACAGACACTGCTGGTCCCTGTGTCTGAGCCTGC ATGTTTGATGGTGTCTGGATGCAAGCAGAAGGGGTGGA |
| | | AGAGCTTGCCTGGAGAGATACAGCTGGGTCAGTAGGAC TGGGACAGGCAGCTGGAGAATTGCCATGTAGATGTTCA TACAATCGTCAAATCATGAAGGCTGGAAAAGCCCTCCA AGATCCCCAAGACCAACCCCAACCCACCCACCGTGCCC ACTGGCCATGTCCCTCAGTGCCACATCCCCACAGTTCTT CATCACCTCCAGGGACGGTGACCCCCCCACCTCCGTGG GCAGCTGTGCCACTGCAGCACCGCTCTTTGGAGAAGGT AAATCTTGCTAAATCCAGCCCGACCCTCCCCTGGCACAA CGTAAGGCCATTATCTCTCATCCAACTCCAGGACGGAGT CAGTGAGAATATTGGCGCGCCGAATTCTCGCGACGATC GGTCGACATAACTTCGTATAGGATACTTTATACGAAGTT ATCAATTCAGGCTAGTTATTAATAGTAATCAATTACGGG GTCATTAGTTCATAGCCCATATATGGAGTTCCGCGTTAC ATAACTTACGGTAAATGGCCCGCCTGGCTGACCGCCCA ACGACCCCCGCCCATTGACGTCAATAATGACGTATGTTC CCATAGTAACGCCAATAGGGACTTTCCATTGACGTCAAT GGGTGGAGTATTTACGGTAAACTGCCCACTTGGCAGTA CATCAAGTGTATCATATGCCAAGTACGCCCCCTATTGAC GTCAATGACGGTAAATGGCCCGCCTGGCATTATGCCCA GTACATGACCTTATGGGACTTTCCTACTTGGCAGTACAT CTACGTATTAGTCATCGCTATTACCATGGTCGAGGTGAG CCCCACGTTCTGCTTCACTCTCCCCATCTCCCCCCCCTCC CCACCCCCAATTTTGTATTTATTTATTTTTTAATTATTTT GTGCAGCGATGGGGGCGGGGGGGGGGGGGGGGCGCGC GCCAGGCGGGGCGGGGCGGGGCGAGGGGCGGGGCGGG GCGAGGCGGAGAGGTGCGGCGGCAGCCAATCAGAGCG GCGCGCTCCGAAAGTTTCCTTTTATGGCGAGGCGGCGGC GGCGGCGGCCCTATAAAAAGCGAAGCGCGCGGCGGGCG GGGAGTCGCTGCGACGCTGCCTTCGCCCCGTGCCCCGCT CCGCCGCCGCCTCGCGCCGCCCGCCCCGGCTCTGACTGA CCGCGTTACTCCCACAGGTGAGCGGGCGGGACGGCCCT TCTCCTCCGGGCTGTAATTAGCGCTTGGTTTAATGACGG CTTGTTTCTTTTCTGTGGCTGCGTGAAAGCCTTGAGGGG CTCCGGGAGGGCCCTTTGTGCGGGGGGAGCGGCTCGGG GGGTGCGTGCGTGTGTGTGTGCGTGGGGAGCGCCGCGT GCGGCTCCGCGCTGCCCGGCGGCTGTGAGCGCTGCGGG CGCGGCGCGGGGCTTTGTGCGCTCCGCAGTGTGCGCGA GGGGAGCGCGGCCGGGGGCGGTGCCCCGCGGTGCGGGG GGGGCTGCGAGGGGAACAAAGGCTGCGTGCGGGGTGTG TGCGTGGGGGGGTGAGCAGGGGGTGTGGGCGCGTCGGT CGGGCTGCAACCCCCCCTGCACCCCCCTCCCCGAGTTGC TGAGCACGGCCCGGCTTCGGGTGCGGGGCTCCGTACGG GGCGTGGCGCGGGGCTCGCCGTGCCGGGCGGGGGGTGG CGGCAGGTGGGGGTGCCGGGCGGGGCGGGGCCGCCTCG GGCCGGGGAGGGCTCGGGGGAGGGGCGCGGCGGCCCC CGGAGCGCCGGCGGCTGTCGAGGCGCGGCGAGCCGCAG CCATTGCCTTTTATGGTAATCGTGCGAGAGGGCGCAGG GACTTCCTTTGTCCCAAATCTGTGCGGAGCCGAAATCTG GGAGGCGCCGCCGCACCCCCTCTAGCGGGCGCGGGGCG AAGCGGTGCGGCGCCGGCAGGAAGGAAATGGGCGGGG AGGGCCTTCGTGCGTCGCCGCGCCGCCGTCCCCTTCTCC CTCTCCAGCCTCGGGGCTGTCCGCGGGGGGACGGCTGC CTTCGGGGGGGACGGGGCAGGGCGGGGTTCGGCTTCTG GCGTGTGACCGGCGGCTCTAGAGCCTCTGCTAACCATGT |
| | | TCATGCCTTCTTCTTTTTCCTACAGCTCCTGGGCAACGTG CTGGTTATTGTGCTGTCTCATCATTTTGGCAAAGAATTG TGTACAACCATGGAGAGCGACGAGAGCGGCCTGCCCGC CATGGAGATCGAGTGCCGCATCACCGGCACCCTGAACG GCGTGGAGTTCGAGCTGGTGGGCGGCGGAGAGGGCACC CCCAAGCAGGGCCGCATGACCAACAAGATGAAGAGCAC CAAAGGCGCCCTGACCTTCAGCCCCTACCTGCTGAGCCA CGTGATGGGCTACGGCTTCTACCACTTCGGCACCTACCC CAGCGGCTACGAGAACCCCTTCCTGCACGCCATCAACA ACGGCGGCTACACCAACACCCGCATCGAGAAGTACGAG GACGGCGGCGTGCTGCACGTGAGCTTCAGCTACCGCTA CGAGGCCGGCCGCGTGATCGGCGACTTCAAGGTGGTGG GCACCGGCTTCCCCGAGGACAGCGTGATCTTCACCGAC AAGATCATCCGCAGCAACGCCACCGTGGAGCACCTGCA CCCCATGGGCGATAACGTGCTGGTGGGCAGCTTCGCCC GCACCTTCAGCCTGCGCGACGGCGGCTACTACAGCTTCG TGGTGGACAGCCACATGCACTTCAAGAGCGCCATCCAC CCCAGCATCCTGCAGAACGGGGGCCCCATGTTCGCCTTC CGCCGCGTGGAGGAGCTGCACAGCAACACCGAGCTGGG CATCGTGGAGTACCAGCACGCCTTCAAGACCCCCATCG CCTTCGCCAGATCCCGCGCTCAGTCGTCCAATTCTGCCG TGGACGGCACCGCCGGACCCGGCTCCACCGGATCTCGC TAAACGCGTGAATTCACTCCTCAGGTGCAGGCTGCCTAT CAGAAGGTGGTGGCTGGTGTGGCCAATGCCCTGGCTCA CAAATACCACTGAGATCTTTTTCCCTCTGCCAAAAATTA TGGGGACATCATGAAGCCCCTTGAGCATCTGACTTCTGG CTAATAAAGGAAATTTATTTTCATTGCAATAGTGTGTTG GAATTTTTTGTGTCTCTCACTCGGAAGGACATATGGGAG GGCAAATCATTTAAAACATCAGAATGAGTATTTGGTTTA GAGTTTGGCAACATATGCCCATATGCTGGCTGCCATGAA CAAAGGTTGGCTATAAAGAGGTCATCAGTATATGAAAC AGCCCCCTGCTGTCCATTCCTTATTCCATAGAAAAGCCT TGACTTGAGGTTAGATTTTTTTTATATTTTGTTTTGTGTT ATTTTTTTCTTTAACATCCCTAAAATTTTCCTTACATGTT TTACTAGCCAGATTTTTCCTCCTCTCCTGACTACTCCCAG TCATAGCTGTCCCTCTTCTCTTATGGAGATCACCGGTAT AACTTCGTATAATGTATACTATACGAAGTTATGGTACCT TAATTAAGTTAACATGCATAGTACTGAGCTCACGGGGA CAGCCCCCCCCCAAAGCCCCCAGGGATGTAATTACGTC CCTCCCCCGCTAGGGGGCAGCAGCGAGCCGCCCGGGGC TCCGCTCCGGTCCGGCGCTCCCCCCGCATCCCCGAGCCG GCAGCGTGCGGGGACAGCCCGGGCACGGGGAAGGTGG CACGGGATCGCTTTCCTCTGAACGCTTCTCGCTGCTCTTT GAGCCTGCAGACACCTGGGGGGATACGGGGAAAAAGCT TTAGGCTGAAAGAGAGATTTAGAATGACAGAATCATAG AACGGCCTGGGTTGCAAAGGAGCACAGTGCTCATCCAG ATCCAACCCCCTGCTATGTGCAGGGTCATCAACCAGCA GCCCAGGCTGCCCAGAGCCACATCCAGCCTGGCCTTGA ATGCCTGCAGGGATGGGGCATCCACAGCCTCCTTGGGC AACCTGTTCAGTGCGTCACCACCCTCTGGGGGAAAAAC TGCCTCCTCATATCCAACCCAAACCTCCCCTGTCTCAGT GTAAAGCCATTCCCCCTTGTCCTATCAAGGGGGAGTTTG CTGTGACATTGTTGGTCTGGGGTGACACATGTTTGCCAA TTCAGTGCATCACGGAGAGGCAGATCTTGGGGATAAGG |
| | | AAGTGCAGGACAGCATGGACGTGGGACATGCAGGTGTT GAGGGCTCTGGGACACTCTCCAAGTCACAGCGTTCAGA ACAGCCTTAAGGATAAGAAGATAGGATAGAAGGACAA AGAGCAAGTTAAAACCCAGCATGGAGAGGAGCACAAA AAGGCCACAGACACTGCTGGTCCCTGTGTCTGAGCCTGC ATGTTTGATGGTGTCTGGATGCAAGCAGAAGGGGTGGA AGAGCTTGCCTGGAGAGATACAGCTGGGTCAGTAGGAC TGGGACAGGCAGCTGGAGAATTGCCATGTAGATGTTCA TACAATCGTCAAATCATGAAGGCTGGAAAAGCCCTCCA AGATCCCCAAGACCAACCCCAACCCACCCACCGTGCCC ACTGGCCATGTCCCTCAGTGCCACATCCCCACAGTTCTT CATCACCTCCAGGGACGGTGACCCCCCCACCTCCGTGG GCAGCTGTGCCACTGCAGCACCGCTCTTTGGAGAAGGT AAATCTTGCTAAATCCAGCCCGACCCTCCCCTGGCACAA CGTAAGGCCATTATCTCTCATCCAACTCCAGGACGGAGT CAGTGAGAATATTGTTTAAACTCTAGGCCAAATCTGGCC TCCCAATTGTTTTATAAATAAAATTTTATTTAAACGTAA CCACTCCCATTCATTTGCTTGTCTCTGGTTGTTTTTGCAA TACAACAGTAGGGTTAGGTAGATAATACAGGCTGTGTG TCACATGATGAAGCCAAAAACATTTGCTCTCTGGCTCTT TACAGAATGAATTTGCTTATATTTCTTCTAGGATGTCAA TTTGTCTGCTTAATTCACAGAAGGACAAATTTTAACATA ACTTACTTCTATGGCCTGAATGTTTGCGTACACTGAAAA TTGATTTGTTGAAATCGATCCCCAATTTGATAGTATTAG GAGGTAGGGCTTTGGAGATGTGATTAGATCATGAGGGT GTAACCTTTATTAATGGGATTAGTGCTTTTATATGGGGT TGAAGAGACCCGAGCTCCCCTTCCACCTTGTGAGGATGC AGAAAGAAGATACATTCTGTGAACTGGAAAGCCGGCCC TCACTAGACACCAAATATGCCAGTATTTTGATCTTGGGC TTCCCAGCCTTCAGAACTTACAGCAGTCCAGAAAGATTA AGACACCCACCTGAAATGTTAATTTTGTCCTAAAATAAT CTTGCATTTAAGATCTGAAATATGACCTAAGTATTATCT GAGGAGTCAATGCTGATTTATTGGTGGTGGATACTGTGA TTCTGTATTAAGAATGGATCTGAAGCCACATAAGATAA CTGTGATTACTTAGTGCTATTTTCAATGGCTGTGAGAAG ACAACCTTGTGACCAGAATTTGAAACCCTGCATAAACA AAACTCAACATGTTTTGAAGGCCTTTACACTCTTCCTCT CAGGGTTTAAACACTGGCCGTCGTTTTACAACGTCGTGA CTGGGAAAACCCTGGCGTTACCCAACTTAATCGCCTTGC AGCACATCCCCCTTTCGCCAGCTGGCGTAATAGCGAAG AGGCCCGCACCGATCGCCCTTCCCAACAGTTGCGCAGC CTGAATGGCGAATGGCGCCTGATGCGGTATTTTCTCCTT ACGCATCTGTGCGGTATTTCACACCGCATATGGTGCACT CTCAGTACAATCTGCTCTGATGCCGCATAGTTAAGCCAG CCCCGACACCCGCCAACACCCGCTGACGCGCCCTGACG GGCTTGTCTGCTCCCGGCATCCGCTTACAGACAAGCTGT GACCGTCTCCGGGAGCTGCATGTGTCAGAGGTTTTCACC GTCATCACCGAAACGCGCGA |
| 24 | GSH3 donor vector | TGCAGCTCTGGCCCGTGTCTCAAAATCTCTGATGTTACA TTGCACAAGATAAAAATATATCATCATGAACAATAAAA CTGTCTGCTTACATAAACAGTAATACAAGGGGTGTTATG AGCCATATTCAACGGGAAACGTCGAGGCCGCGATTAAA TTCCAACATGGATGCTGATTTATATGGGTATAAATGGGC TCGCGATAATGTCGGGCAATCAGGTGCGACAATCTATC |
| | | GCTTGTATGGGAAGCCCGATGCGCCAGAGTTGTTTCTGA AACATGGCAAAGGTAGCGTTGCCAATGATGTTACAGAT GAGATGGTCAGACTAAACTGGCTGACGGAATTTATGCC TCTTCCGACCATCAAGCATTTTATCCGTACTCCTGATGA TGCATGGTTACTCACCACTGCGATCCCCGGAAAAACAG CATTCCAGGTATTAGAAGAATATCCTGATTCAGGTGAA AATATTGTTGATGCGCTGGCAGTGTTCCTGCGCCGGTTG CATTCGATTCCTGTTTGTAATTGTCCTTTTAACAGCGATC GCGTATTTCGTCTCGCTCAGGCGCAATCACGAATGAATA ACGGTTTGGTTGATGCGAGTGATTTTGATGACGAGCGTA ATGGCTGGCCTGTTGAACAAGTCTGGAAAGAAATGCAT AAACTTTTGCCATTCTCACCGGATTCAGTCGTCACTCAT GGTGATTTCTCACTTGATAACCTTATTTTTGACGAGGGG AAATTAATAGGTTGTATTGATGTTGGACGAGTCGGAATC GCAGACCGATACCAGGATCTTGCCATCCTATGGAACTG CCTCGGTGAGTTTTCTCCTTCATTACAGAAACGGCTTTTT CAAAAATATGGTATTGATAATCCTGATATGAATAAATTG CAGTTTCATTTGATGCTCGATGAGTTTTTCTAATCAGAA TTGGTTAATTGGTTGTAACATTATTCAGATTGGGCTTGA TTTAAAACTTCATTTTTAATTTAAAAGGATCTAGGTGAA GATCCTTTTTGATAATCTCATGACCAAAATCCCTTAACG TGAGTTTTCGTTCCACTGAGCGTCAGACCCCGTAGAAAA GATCAAAGGATCTTCTTGAGATCCTTTTTTTCTGCGCGT AATCTGCTGCTTGCAAACAAAAAAACCACCGCTACCAG CGGTGGTTTGTTTGCCGGATCAAGAGCTACCAACTCTTT TTCCGAAGGTAACTGGCTTCAGCAGAGCGCAGATACCA AATACTGTTCTTCTAGTGTAGCCGTAGTTAGGCCACCAC TTCAAGAACTCTGTAGCACCGCCTACATACCTCGCTCTG CTAATCCTGTTACCAGTGGCTGCTGCCAGTGGCGATAAG TCGTGTCTTACCGGGTTGGACTCAAGACGATAGTTACCG GATAAGGCGCAGCGGTCGGGCTGAACGGGGGGTTCGTG CACACAGCCCAGCTTGGAGCGAACGACCTACACCGAAC TGAGATACCTACAGCGTGAGCTATGAGAAAGCGCCACG CTTCCCGAAGGGAGAAAGGCGGACAGGTATCCGGTAAG CGGCAGGGTCGGAACAGGAGAGCGCACGAGGGAGCTTC CAGGGGGAAACGCCTGGTATCTTTATAGTCCTGTCGGGT TTCGCCACCTCTGACTTGAGCGTCGATTTTTGTGATGCT CGTCAGGGGGGCGGAGCCTATGGAAAAACGCCAGCAAC GCGGCCTTTTTACGGTTCCTGGCCTTTTGCTGGCCTTTTG CTCACATGTTCTTTCCTGCGTTATCCCCTGATTCTGTGGA TAACCGTATTACCGCCTTTGAGTGAGCTGATACCGCTCG CCGCAGCCGAACGACCGAGCGCAGCGAGTCAGTGAGCG AGGAAGCGGAAGAGCGCCCAATACGCAAACCGCCTCTC CCCGCGCGTTGGCCGATTCATTAATGCAGCTGGCACGAC AGGTTTCCCGACTGGAAAGCGGGCAGTGAGCGCAACGC AATTAATGTGAGTTAGCTCACTCATTAGGCACCCCAGGC TTTACACTTTATGCTTCCGGCTCGTATGTTGTGTGGAATT GTGAGCGGATAACAATTTCACACAGGAAACAGCTATGA CCATGATTACGCCGCGGCCGCGGCTTCCCTCTGTGATGG GTAGATAATTTTGCTTAATATGTAAACAGTCTTTTCAAC ACTCTGTGCTTAAATAATCATTGTTGGTTTGCTTTCTTCT AGTATTCCATATTAAGTAATGACTTGGCTAATGAGAAG GATGCTTGTAAGTATATTAGCATCAAGTTCAAATATAGA GAGATTAAATAACCTTCCCAAGGTCAACACACTGGTCA |
| | | TTGACAAATATGGACCAGATCTCATCTCCAAAACCCCAC ACAATTATACCAGGTTAGCTCATTCTTTCTTCCTAGACA TCTTATTTTCCAGGGAGTAGTGAAGGTCTACAAAACTTT CCTACTAAATAGGAAATATATTTTGTTGTCAGATGTAAG CCCAGAAAAGCAATGGCTAAACTGCTATGTATCAAAAA CTTTGCAAACTTATAACAGTTTAAAATTTACTTCTAGCT ATGTTTAGAGCCCTAAGGAGATAATGGCAAGTCATTTTT TCTTCTTTATACATATGTCATCATTTATGGACAGAAAAT AGTTCTCAATTGTAAAAAAATAGCTCTCTATAGGATTTG CTGAGTTTTTTATGGCTAGAGTTATTAAGTAAAATAAAT GACATCTGAGAGCACTCTGCTTTGTCTTTGGCTTCAAAT AATGTATTTTACTATATTTTTTCATTCTTCGCTGCCATAA GTGGCAGAAAGAAAACCGCTTTTCAAATGGCTAGTGCC TAAAAAGCAAATACTTTGTGGATCTTGGGGCCAGTGTA AACTCAGAATGCAAGATATCTTCCTCAGCTTCAGTACTT GCACAATGCCTGTCTTTATTAAACATGAGTGATTTGCAA ATATGATGTTTCTGAGACAGCTAATAGCGGCCGCATAA CTTCGTATAGCATACATTATACGAAGTTATCTGACCTCT TCTCTTCCTCCCACAGGGCCTCGAGAGATCTGGCAGCGG AGAGGGCAGAGGAAGTCTTCTAACATGCGGTGACGTGG AGGAGAATCCCGGCCCTAGGATGACCGAGTACAAGCCC ACGGTGCGCCTCGCCACCCGCGACGACGTCCCCAGGGC CGTACGCACCCTCGCCGCCGCGTTCGCCGACTACCCCGC CACGCGCCACACCGTCGATCCGGACCGCCACATCGAGC GGGTCACCGAGCTGCAAGAACTCTTCCTCACGCGCGTC GGGCTCGACATCGGCAAGGTGTGGGTCGCGGACGACGG CGCCGCGGTGGCGGTCTGGACCACGCCGGAGAGCGTCG AAGCGGGGGCGGTGTTCGCCGAGATCGGCCCGCGCATG GCCGAGTTGAGCGGTTCCCGGCTGGCCGCGCAGCAACA GATGGAAGGCCTCCTGGCGCCGCACCGGCCCAAGGAGC CCGCGTGGTTCCTGGCCACCGTCGGCGTCTCGCCCGACC ACCAGGGCAAGGGTCTGGGCAGCGCCGTCGTGCTCCCC GGAGTGGAGGCGGCCGAGCGCGCCGGGGTGCCCGCCTT CCTGGAGACCTCCGCGCCCCGCAACCTCCCCTTCTACGA GCGGCTCGGCTTCACCGTCACCGCCGACGTCGAGGTGC CCGAAGGACCGCGCACCTGGTGCATGACCCGCAAGCCC GGTGCCTGACTGTGCCTTCTAGTTGCCAGCCATCTGTTG TTTGCCCCTCCCCCGTGCCTTCCTTGACCCTGGAAGGTG CCACTCCCACTGTCCTTTCCTAATAAAATGAGGAAATTG CATCGCATTGTCTGAGTAGGTGTCATTCTATTCTGGGGG GTGGGGTGGGGCAGGACAGCAAGGGGGAGGATTGGGA AGACAATAGCAGGCATGCTGGGGATGCGGTGGGCTCTA TGGATAACTTCGTATAGCATACATTATACGAAGTTATGA GCTCACGGGGACAGCCCCCCCCCAAAGCCCCCAGGGAT GTAATTACGTCCCTCCCCCGCTAGGGGGCAGCAGCGAG CCGCCCGGGGCTCCGCTCCGGTCCGGCGCTCCCCCCGCA TCCCCGAGCCGGCAGCGTGCGGGGACAGCCCGGGCACG GGGAAGGTGGCACGGGATCGCTTTCCTCTGAACGCTTCT CGCTGCTCTTTGAGCCTGCAGACACCTGGGGGGATACG GGGAAAAAGCTTTAGGCTGAAAGAGAGATTTAGAATGA CAGAATCATAGAACGGCCTGGGTTGCAAAGGAGCACAG TGCTCATCCAGATCCAACCCCCTGCTATGTGCAGGGTCA TCAACCAGCAGCCCAGGCTGCCCAGAGCCACATCCAGC CTGGCCTTGAATGCCTGCAGGGATGGGGCATCCACAGC |
| | | CTCCTTGGGCAACCTGTTCAGTGCGTCACCACCCTCTGG GGGAAAAACTGCCTCCTCATATCCAACCCAAACCTCCCC TGTCTCAGTGTAAAGCCATTCCCCCTTGTCCTATCAAGG GGGAGTTTGCTGTGACATTGTTGGTCTGGGGTGACACAT GTTTGCCAATTCAGTGCATCACGGAGAGGCAGATCTTG GGGATAAGGAAGTGCAGGACAGCATGGACGTGGGACAT GCAGGTGTTGAGGGCTCTGGGACACTCTCCAAGTCACA GCGTTCAGAACAGCCTTAAGGATAAGAAGATAGGATAG AAGGACAAAGAGCAAGTTAAAACCCAGCATGGAGAGG AGCACAAAAAGGCCACAGACACTGCTGGTCCCTGTGTC TGAGCCTGCATGTTTGATGGTGTCTGGATGCAAGCAGA AGGGGTGGAAGAGCTTGCCTGGAGAGATACAGCTGGGT CAGTAGGACTGGGACAGGCAGCTGGAGAATTGCCATGT AGATGTTCATACAATCGTCAAATCATGAAGGCTGGAAA AGCCCTCCAAGATCCCCAAGACCAACCCCAACCCACCC ACCGTGCCCACTGGCCATGTCCCTCAGTGCCACATCCCC ACAGTTCTTCATCACCTCCAGGGACGGTGACCCCCCCAC CTCCGTGGGCAGCTGTGCCACTGCAGCACCGCTCTTTGG AGAAGGTAAATCTTGCTAAATCCAGCCCGACCCTCCCCT GGCACAACGTAAGGCCATTATCTCTCATCCAACTCCAGG ACGGAGTCAGTGAGAATATTGGCGCGCCGAATTCTCGC GACGATCGGTCGACATAACTTCGTATAGGATACTTTATA CGAAGTTATCAATTCAGGCTAGTTATTAATAGTAATCAA TTACGGGGTCATTAGTTCATAGCCCATATATGGAGTTCC GCGTTACATAACTTACGGTAAATGGCCCGCCTGGCTGAC CGCCCAACGACCCCCGCCCATTGACGTCAATAATGACG TATGTTCCCATAGTAACGCCAATAGGGACTTTCCATTGA CGTCAATGGGTGGAGTATTTACGGTAAACTGCCCACTTG GCAGTACATCAAGTGTATCATATGCCAAGTACGCCCCCT ATTGACGTCAATGACGGTAAATGGCCCGCCTGGCATTAT GCCCAGTACATGACCTTATGGGACTTTCCTACTTGGCAG TACATCTACGTATTAGTCATCGCTATTACCATGGTCGAG GTGAGCCCCACGTTCTGCTTCACTCTCCCCATCTCCCCC CCCTCCCCACCCCCAATTTTGTATTTATTTATTTTTTAAT TATTTTGTGCAGCGATGGGGGCGGGGGGGGGGGGGGGG CGCGCGCCAGGCGGGGCGGGGCGGGGCGAGGGGCGGG GCGGGGCGAGGCGGAGAGGTGCGGCGGCAGCCAATCA GAGCGGCGCGCTCCGAAAGTTTCCTTTTATGGCGAGGC GGCGGCGGCGGCGGCCCTATAAAAAGCGAAGCGCGCGG CGGGCGGGGAGTCGCTGCGACGCTGCCTTCGCCCCGTG CCCCGCTCCGCCGCCGCCTCGCGCCGCCCGCCCCGGCTC TGACTGACCGCGTTACTCCCACAGGTGAGCGGGCGGGA CGGCCCTTCTCCTCCGGGCTGTAATTAGCGCTTGGTTTA ATGACGGCTTGTTTCTTTTCTGTGGCTGCGTGAAAGCCT TGAGGGGCTCCGGGAGGGCCCTTTGTGCGGGGGGAGCG GCTCGGGGGGTGCGTGCGTGTGTGTGTGCGTGGGGAGC GCCGCGTGCGGCTCCGCGCTGCCCGGCGGCTGTGAGCG CTGCGGGCGCGGCGCGGGGCTTTGTGCGCTCCGCAGTG TGCGCGAGGGGAGCGCGGCCGGGGGCGGTGCCCCGCGG TGCGGGGGGGGCTGCGAGGGGAACAAAGGCTGCGTGCG GGGTGTGTGCGTGGGGGGGTGAGCAGGGGGTGTGGGCG CGTCGGTCGGGCTGCAACCCCCCCTGCACCCCCCTCCCC GAGTTGCTGAGCACGGCCCGGCTTCGGGTGCGGGGCTC CGTACGGGGCGTGGCGCGGGGCTCGCCGTGCCGGGCGG |
| | | GGGGTGGCGGCAGGTGGGGGTGCCGGGCGGGGCGGGG CCGCCTCGGGCCGGGGAGGGCTCGGGGGAGGGGCGCGG CGGCCCCCGGAGCGCCGGCGGCTGTCGAGGCGCGGCGA GCCGCAGCCATTGCCTTTTATGGTAATCGTGCGAGAGGG CGCAGGGACTTCCTTTGTCCCAAATCTGTGCGGAGCCGA AATCTGGGAGGCGCCGCCGCACCCCCTCTAGCGGGCGC GGGGCGAAGCGGTGCGGCGCCGGCAGGAAGGAAATGG GCGGGGAGGGCCTTCGTGCGTCGCCGCGCCGCCGTCCC CTTCTCCCTCTCCAGCCTCGGGGCTGTCCGCGGGGGGAC GGCTGCCTTCGGGGGGGACGGGGCAGGGCGGGGTTCGG CTTCTGGCGTGTGACCGGCGGCTCTAGAGCCTCTGCTAA CCATGTTCATGCCTTCTTCTTTTTCCTACAGCTCCTGGGC AACGTGCTGGTTATTGTGCTGTCTCATCATTTTGGCAAA GAATTGTGTACAACCATGGAGAGCGACGAGAGCGGCCT GCCCGCCATGGAGATCGAGTGCCGCATCACCGGCACCC TGAACGGCGTGGAGTTCGAGCTGGTGGGCGGCGGAGAG GGCACCCCCAAGCAGGGCCGCATGACCAACAAGATGAA GAGCACCAAAGGCGCCCTGACCTTCAGCCCCTACCTGCT GAGCCACGTGATGGGCTACGGCTTCTACCACTTCGGCAC CTACCCCAGCGGCTACGAGAACCCCTTCCTGCACGCCAT CAACAACGGCGGCTACACCAACACCCGCATCGAGAAGT ACGAGGACGGCGGCGTGCTGCACGTGAGCTTCAGCTAC CGCTACGAGGCCGGCCGCGTGATCGGCGACTTCAAGGT GGTGGGCACCGGCTTCCCCGAGGACAGCGTGATCTTCA CCGACAAGATCATCCGCAGCAACGCCACCGTGGAGCAC CTGCACCCCATGGGCGATAACGTGCTGGTGGGCAGCTT CGCCCGCACCTTCAGCCTGCGCGACGGCGGCTACTACA GCTTCGTGGTGGACAGCCACATGCACTTCAAGAGCGCC ATCCACCCCAGCATCCTGCAGAACGGGGGCCCCATGTT CGCCTTCCGCCGCGTGGAGGAGCTGCACAGCAACACCG AGCTGGGCATCGTGGAGTACCAGCACGCCTTCAAGACC CCCATCGCCTTCGCCAGATCCCGCGCTCAGTCGTCCAAT TCTGCCGTGGACGGCACCGCCGGACCCGGCTCCACCGG ATCTCGCTAAACGCGTGAATTCACTCCTCAGGTGCAGGC TGCCTATCAGAAGGTGGTGGCTGGTGTGGCCAATGCCCT GGCTCACAAATACCACTGAGATCTTTTTCCCTCTGCCAA AAATTATGGGGACATCATGAAGCCCCTTGAGCATCTGA CTTCTGGCTAATAAAGGAAATTTATTTTCATTGCAATAG TGTGTTGGAATTTTTTGTGTCTCTCACTCGGAAGGACAT ATGGGAGGGCAAATCATTTAAAACATCAGAATGAGTAT TTGGTTTAGAGTTTGGCAACATATGCCCATATGCTGGCT GCCATGAACAAAGGTTGGCTATAAAGAGGTCATCAGTA TATGAAACAGCCCCCTGCTGTCCATTCCTTATTCCATAG AAAAGCCTTGACTTGAGGTTAGATTTTTTTTATATTTTGT TTTGTGTTATTTTTTTCTTTAACATCCCTAAAATTTTCCTT ACATGTTTTACTAGCCAGATTTTTCCTCCTCTCCTGACTA CTCCCAGTCATAGCTGTCCCTCTTCTCTTATGGAGATCA CCGGTATAACTTCGTATAATGTATACTATACGAAGTTAT GGTACCTTAATTAAGTTAACATGCATAGTACTGAGCTCA CGGGGACAGCCCCCCCCCAAAGCCCCCAGGGATGTAAT TACGTCCCTCCCCCGCTAGGGGGCAGCAGCGAGCCGCC CGGGGCTCCGCTCCGGTCCGGCGCTCCCCCCGCATCCCC GAGCCGGCAGCGTGCGGGGACAGCCCGGGCACGGGGA AGGTGGCACGGGATCGCTTTCCTCTGAACGCTTCTCGCT |
| | | GCTCTTTGAGCCTGCAGACACCTGGGGGGATACGGGGA AAAAGCTTTAGGCTGAAAGAGAGATTTAGAATGACAGA ATCATAGAACGGCCTGGGTTGCAAAGGAGCACAGTGCT CATCCAGATCCAACCCCCTGCTATGTGCAGGGTCATCAA CCAGCAGCCCAGGCTGCCCAGAGCCACATCCAGCCTGG CCTTGAATGCCTGCAGGGATGGGGCATCCACAGCCTCCT TGGGCAACCTGTTCAGTGCGTCACCACCCTCTGGGGGA AAAACTGCCTCCTCATATCCAACCCAAACCTCCCCTGTC TCAGTGTAAAGCCATTCCCCCTTGTCCTATCAAGGGGGA GTTTGCTGTGACATTGTTGGTCTGGGGTGACACATGTTT GCCAATTCAGTGCATCACGGAGAGGCAGATCTTGGGGA TAAGGAAGTGCAGGACAGCATGGACGTGGGACATGCAG GTGTTGAGGGCTCTGGGACACTCTCCAAGTCACAGCGTT CAGAACAGCCTTAAGGATAAGAAGATAGGATAGAAGG ACAAAGAGCAAGTTAAAACCCAGCATGGAGAGGAGCA CAAAAAGGCCACAGACACTGCTGGTCCCTGTGTCTGAG CCTGCATGTTTGATGGTGTCTGGATGCAAGCAGAAGGG GTGGAAGAGCTTGCCTGGAGAGATACAGCTGGGTCAGT AGGACTGGGACAGGCAGCTGGAGAATTGCCATGTAGAT GTTCATACAATCGTCAAATCATGAAGGCTGGAAAAGCC CTCCAAGATCCCCAAGACCAACCCCAACCCACCCACCG TGCCCACTGGCCATGTCCCTCAGTGCCACATCCCCACAG TTCTTCATCACCTCCAGGGACGGTGACCCCCCCACCTCC GTGGGCAGCTGTGCCACTGCAGCACCGCTCTTTGGAGA AGGTAAATCTTGCTAAATCCAGCCCGACCCTCCCCTGGC ACAACGTAAGGCCATTATCTCTCATCCAACTCCAGGACG GAGTCAGTGAGAATATTGTTTAAACTCATGGGCCAAAA TGAATGGAATCAAAACCTGAGACACATTTCTGTTAATG GGGCTCTATTTGAGAAATGTGCATTATTTTATCAGCTTC CTTATTTTAACCATTCTACCCTGTCAATACTAATACCAA GTGTTCATTTTCTTTATTAGTTTAAATGGGAGAAAACAA TGCAGAAAGCTTAATAACTGTTCTATTTTTAAAGCCTTT ATAGAAAACATAATATAAAAAGTGATATGAACTAGGCA CAAGACAAAACTGACTTTAATATGTTATATGTTAATCTG TTAAAATACAGTACATTTTACTGCAGAATGTTAGCAGGG TATATTTGTAGATAATAGATCATTGACATTTTTATCCTAT ATCCTTTTTACCTTTTATTCCTTTTAAACATTGATTACCT TTGTTATAAAAAGCAACAACAATAGTTTGTAAAAGATT AAGATATTTAAATATTTAAAAATTTTTCATAACTATATT AGTAAACAGTAGTACATAAGTCTTGATAGGTACAAATG ATGTCTCAAAAGACAGATGAGGTGGAAAATTACCACTC TAGCTCATGGCATGACCCCAGCCGTGCAGAGGAAAGAT TTCTTCTCCTCCCCTATTCACCTCCGGGTCCCAGCTATCA CTAAATATATCGGGGGTCGCGTTGCTGATCAGCAGCCTT AAGCCTGAACATGGTGGGTCCTTTGCATTAGTTCTCAAT TCCCACCACCCTCCTCCCTTCCTGTGTCACGAGGCTCAC TCATTTCCTCAGTGCCCTTCCCAGTTACTGTCAAATCAT ATAAACTCATTTCTCTGCCTCCAGCCTTTTATTTCATCGT TTAAACACTGGCCGTCGTTTTACAACGTCGTGACTGGGA AAACCCTGGCGTTACCCAACTTAATCGCCTTGCAGCACA TCCCCCTTTCGCCAGCTGGCGTAATAGCGAAGAGGCCC GCACCGATCGCCCTTCCCAACAGTTGCGCAGCCTGAATG GCGAATGGCGCCTGATGCGGTATTTTCTCCTTACGCATC TGTGCGGTATTTCACACCGCATATGGTGCACTCTCAGTA |
| | | CAATCTGCTCTGATGCCGCATAGTTAAGCCAGCCCCGAC ACCCGCCAACACCCGCTGACGCGCCCTGACGGGCTTGT CTGCTCCCGGCATCCGCTTACAGACAAGCTGTGACCGTC TCCGGGAGCTGCATGTGTCAGAGGTTTTCACCGTCATCA CCGAAACGCGCGA |
| 25 | AAVS1 donor vector | TGCAGCTCTGGCCCGTGTCTCAAAATCTCTGATGTTACA TTGCACAAGATAAAAATATATCATCATGAACAATAAAA CTGTCTGCTTACATAAACAGTAATACAAGGGGTGTTATG AGCCATATTCAACGGGAAACGTCGAGGCCGCGATTAAA TTCCAACATGGATGCTGATTTATATGGGTATAAATGGGC TCGCGATAATGTCGGGCAATCAGGTGCGACAATCTATC GCTTGTATGGGAAGCCCGATGCGCCAGAGTTGTTTCTGA AACATGGCAAAGGTAGCGTTGCCAATGATGTTACAGAT GAGATGGTCAGACTAAACTGGCTGACGGAATTTATGCC TCTTCCGACCATCAAGCATTTTATCCGTACTCCTGATGA TGCATGGTTACTCACCACTGCGATCCCCGGAAAAACAG CATTCCAGGTATTAGAAGAATATCCTGATTCAGGTGAA AATATTGTTGATGCGCTGGCAGTGTTCCTGCGCCGGTTG CATTCGATTCCTGTTTGTAATTGTCCTTTTAACAGCGATC GCGTATTTCGTCTCGCTCAGGCGCAATCACGAATGAATA ACGGTTTGGTTGATGCGAGTGATTTTGATGACGAGCGTA ATGGCTGGCCTGTTGAACAAGTCTGGAAAGAAATGCAT AAACTTTTGCCATTCTCACCGGATTCAGTCGTCACTCAT GGTGATTTCTCACTTGATAACCTTATTTTTGACGAGGGG AAATTAATAGGTTGTATTGATGTTGGACGAGTCGGAATC GCAGACCGATACCAGGATCTTGCCATCCTATGGAACTG CCTCGGTGAGTTTTCTCCTTCATTACAGAAACGGCTTTTT CAAAAATATGGTATTGATAATCCTGATATGAATAAATTG CAGTTTCATTTGATGCTCGATGAGTTTTTCTAATCAGAA TTGGTTAATTGGTTGTAACATTATTCAGATTGGGCTTGA TTTAAAACTTCATTTTTAATTTAAAAGGATCTAGGTGAA GATCCTTTTTGATAATCTCATGACCAAAATCCCTTAACG TGAGTTTTCGTTCCACTGAGCGTCAGACCCCGTAGAAAA GATCAAAGGATCTTCTTGAGATCCTTTTTTTCTGCGCGT AATCTGCTGCTTGCAAACAAAAAAACCACCGCTACCAG CGGTGGTTTGTTTGCCGGATCAAGAGCTACCAACTCTTT TTCCGAAGGTAACTGGCTTCAGCAGAGCGCAGATACCA AATACTGTTCTTCTAGTGTAGCCGTAGTTAGGCCACCAC TTCAAGAACTCTGTAGCACCGCCTACATACCTCGCTCTG CTAATCCTGTTACCAGTGGCTGCTGCCAGTGGCGATAAG TCGTGTCTTACCGGGTTGGACTCAAGACGATAGTTACCG GATAAGGCGCAGCGGTCGGGCTGAACGGGGGGTTCGTG CACACAGCCCAGCTTGGAGCGAACGACCTACACCGAAC TGAGATACCTACAGCGTGAGCTATGAGAAAGCGCCACG CTTCCCGAAGGGAGAAAGGCGGACAGGTATCCGGTAAG CGGCAGGGTCGGAACAGGAGAGCGCACGAGGGAGCTTC CAGGGGGAAACGCCTGGTATCTTTATAGTCCTGTCGGGT TTCGCCACCTCTGACTTGAGCGTCGATTTTTGTGATGCT CGTCAGGGGGGCGGAGCCTATGGAAAAACGCCAGCAAC GCGGCCTTTTTACGGTTCCTGGCCTTTTGCTGGCCTTTTG CTCACATGTTCTTTCCTGCGTTATCCCCTGATTCTGTGGA TAACCGTATTACCGCCTTTGAGTGAGCTGATACCGCTCG CCGCAGCCGAACGACCGAGCGCAGCGAGTCAGTGAGCG AGGAAGCGGAAGAGCGCCCAATACGCAAACCGCCTCTC |
| | | CCCGCGCGTTGGCCGATTCATTAATGCAGCTGGCACGAC AGGTTTCCCGACTGGAAAGCGGGCAGTGAGCGCAACGC AATTAATGTGAGTTAGCTCACTCATTAGGCACCCCAGGC TTTACACTTTATGCTTCCGGCTCGTATGTTGTGTGGAATT GTGAGCGGATAACAATTTCACACAGGAAACAGCTATGA CCATGATTACGCCGCGGCCGCTGCTTTCTCTGACCAGCA TTCTCTCCCCTGGGCCTGTGCCGCTTTCTGTCTGCAGCTT GTGGCCTGGGTCACCTCTACGGCTGGCCCAGATCCTTCC CTGCCGCCTCCTTCAGGTTCCGTCTTCCTCCACTCCCTCT TCCCCTTGCTCTCTGCTGTGTTGCTGCCCAAGGATGCTCT TTCCGGAGCACTTCCTTCTCGGCGCTGCACCACGTGATG TCCTCTGAGCGGATCCTCCCCGTGTCTGGGTCCTCTCCG GGCATCTCTCCTCCCTCACCCAACCCCATGCCGTCTTCA CTCGCTGGGTTCCCTTTTCCTTCTCCTTCTGGGGCCTGTG CCATCTCTCGTTTCTTAGGATGGCCTTCTCCGACGGATG TCTCCCTTGCGTCCCGCCTCCCCTTCTTGTAGGCCTGCAT CATCACCGTTTTTCTGGACAACCCCAAAGTACCCCGTCT CCCTGGCTTTAGCCACCTCTCCATCCTCTTGCTTTCTTTG CCTGGACACCCCGTTCTCCTGTGGATTCGGGTCACCTCT CACTCCTTTCATTTGGGCAGCTCCCCTACCCCCCTTACCT CTCTAGTCTGTGCTAGCTCTTCCAGCCCCCTGTCATGGC ATCTTCCAGGGGTCCGAGAGCTCAGCTAGTCTTCTTCCT CCAACCCGGGCCCCTATGTCCACTTCAGGACAGCATGTT TGCTGCCTCCAGGGATCCTGTGTCCCCGAGCTGGGACCA CCTTATATTCCCAGGGCCGGTTAATGTGGCTCTGGTTCT GGGTACTTTTATCTGTCCCCTCCACCCCACAGTGGGGCG CGGCCGCATAACTTCGTATAGCATACATTATACGAAGTT ATCTGACCTCTTCTCTTCCTCCCACAGGGCCTCGAGAGA TCTGGCAGCGGAGAGGGCAGAGGAAGTCTTCTAACATG CGGTGACGTGGAGGAGAATCCCGGCCCTAGGATGACCG AGTACAAGCCCACGGTGCGCCTCGCCACCCGCGACGAC GTCCCCAGGGCCGTACGCACCCTCGCCGCCGCGTTCGCC GACTACCCCGCCACGCGCCACACCGTCGATCCGGACCG CCACATCGAGCGGGTCACCGAGCTGCAAGAACTCTTCC TCACGCGCGTCGGGCTCGACATCGGCAAGGTGTGGGTC GCGGACGACGGCGCCGCGGTGGCGGTCTGGACCACGCC GGAGAGCGTCGAAGCGGGGGCGGTGTTCGCCGAGATCG GCCCGCGCATGGCCGAGTTGAGCGGTTCCCGGCTGGCC GCGCAGCAACAGATGGAAGGCCTCCTGGCGCCGCACCG GCCCAAGGAGCCCGCGTGGTTCCTGGCCACCGTCGGCG TCTCGCCCGACCACCAGGGCAAGGGTCTGGGCAGCGCC GTCGTGCTCCCCGGAGTGGAGGCGGCCGAGCGCGCCGG GGTGCCCGCCTTCCTGGAGACCTCCGCGCCCCGCAACCT CCCCTTCTACGAGCGGCTCGGCTTCACCGTCACCGCCGA CGTCGAGGTGCCCGAAGGACCGCGCACCTGGTGCATGA CCCGCAAGCCCGGTGCCTGACTGTGCCTTCTAGTTGCCA GCCATCTGTTGTTTGCCCCTCCCCCGTGCCTTCCTTGACC CTGGAAGGTGCCACTCCCACTGTCCTTTCCTAATAAAAT GAGGAAATTGCATCGCATTGTCTGAGTAGGTGTCATTCT ATTCTGGGGGGTGGGGTGGGGCAGGACAGCAAGGGGG AGGATTGGGAAGACAATAGCAGGCATGCTGGGGATGCG GTGGGCTCTATGGATAACTTCGTATAGCATACATTATAC GAAGTTATGAGCTCACGGGGACAGCCCCCCCCCAAAGC CCCCAGGGATGTAATTACGTCCCTCCCCCGCTAGGGGGC |
| | | AGCAGCGAGCCGCCCGGGGCTCCGCTCCGGTCCGGCGC TCCCCCCGCATCCCCGAGCCGGCAGCGTGCGGGGACAG CCCGGGCACGGGGAAGGTGGCACGGGATCGCTTTCCTC TGAACGCTTCTCGCTGCTCTTTGAGCCTGCAGACACCTG GGGGGATACGGGGAAAAAGCTTTAGGCTGAAAGAGAG ATTTAGAATGACAGAATCATAGAACGGCCTGGGTTGCA AAGGAGCACAGTGCTCATCCAGATCCAACCCCCTGCTA TGTGCAGGGTCATCAACCAGCAGCCCAGGCTGCCCAGA GCCACATCCAGCCTGGCCTTGAATGCCTGCAGGGATGG GGCATCCACAGCCTCCTTGGGCAACCTGTTCAGTGCGTC ACCACCCTCTGGGGGAAAAACTGCCTCCTCATATCCAAC CCAAACCTCCCCTGTCTCAGTGTAAAGCCATTCCCCCTT GTCCTATCAAGGGGGAGTTTGCTGTGACATTGTTGGTCT GGGGTGACACATGTTTGCCAATTCAGTGCATCACGGAG AGGCAGATCTTGGGGATAAGGAAGTGCAGGACAGCATG GACGTGGGACATGCAGGTGTTGAGGGCTCTGGGACACT CTCCAAGTCACAGCGTTCAGAACAGCCTTAAGGATAAG AAGATAGGATAGAAGGACAAAGAGCAAGTTAAAACCC AGCATGGAGAGGAGCACAAAAAGGCCACAGACACTGCT GGTCCCTGTGTCTGAGCCTGCATGTTTGATGGTGTCTGG ATGCAAGCAGAAGGGGTGGAAGAGCTTGCCTGGAGAGA TACAGCTGGGTCAGTAGGACTGGGACAGGCAGCTGGAG AATTGCCATGTAGATGTTCATACAATCGTCAAATCATGA AGGCTGGAAAAGCCCTCCAAGATCCCCAAGACCAACCC CAACCCACCCACCGTGCCCACTGGCCATGTCCCTCAGTG CCACATCCCCACAGTTCTTCATCACCTCCAGGGACGGTG ACCCCCCCACCTCCGTGGGCAGCTGTGCCACTGCAGCAC CGCTCTTTGGAGAAGGTAAATCTTGCTAAATCCAGCCCG ACCCTCCCCTGGCACAACGTAAGGCCATTATCTCTCATC CAACTCCAGGACGGAGTCAGTGAGAATATTGGCGCGCC GAATTCTCGCGACGATCGGTCGACATAACTTCGTATAGG ATACTTTATACGAAGTTATCAATTCAGGCTAGTTATTAA TAGTAATCAATTACGGGGTCATTAGTTCATAGCCCATAT ATGGAGTTCCGCGTTACATAACTTACGGTAAATGGCCCG CCTGGCTGACCGCCCAACGACCCCCGCCCATTGACGTCA ATAATGACGTATGTTCCCATAGTAACGCCAATAGGGAC TTTCCATTGACGTCAATGGGTGGAGTATTTACGGTAAAC TGCCCACTTGGCAGTACATCAAGTGTATCATATGCCAAG TACGCCCCCTATTGACGTCAATGACGGTAAATGGCCCGC CTGGCATTATGCCCAGTACATGACCTTATGGGACTTTCC TACTTGGCAGTACATCTACGTATTAGTCATCGCTATTAC CATGGTCGAGGTGAGCCCCACGTTCTGCTTCACTCTCCC CATCTCCCCCCCCTCCCCACCCCCAATTTTGTATTTATTT ATTTTTTAATTATTTTGTGCAGCGATGGGGGCGGGGGGG GGGGGGGGGCGCGCGCCAGGCGGGGCGGGGCGGGGCG AGGGGCGGGGCGGGGCGAGGCGGAGAGGTGCGGCGGC AGCCAATCAGAGCGGCGCGCTCCGAAAGTTTCCTTTTAT GGCGAGGCGGCGGCGGCGGCGGCCCTATAAAAAGCGA AGCGCGCGGCGGGCGGGGAGTCGCTGCGACGCTGCCTT CGCCCCGTGCCCCGCTCCGCCGCCGCCTCGCGCCGCCCG CCCCGGCTCTGACTGACCGCGTTACTCCCACAGGTGAGC GGGCGGGACGGCCCTTCTCCTCCGGGCTGTAATTAGCGC TTGGTTTAATGACGGCTTGTTTCTTTTCTGTGGCTGCGTG AAAGCCTTGAGGGGCTCCGGGAGGGCCCTTTGTGCGGG |
| | | GGGAGCGGCTCGGGGGGTGCGTGCGTGTGTGTGTGCGT GGGGAGCGCCGCGTGCGGCTCCGCGCTGCCCGGCGGCT GTGAGCGCTGCGGGCGCGGCGCGGGGCTTTGTGCGCTC CGCAGTGTGCGCGAGGGGAGCGCGGCCGGGGGCGGTGC CCCGCGGTGCGGGGGGGGCTGCGAGGGGAACAAAGGCT GCGTGCGGGGTGTGTGCGTGGGGGGGTGAGCAGGGGGT GTGGGCGCGTCGGTCGGGCTGCAACCCCCCCTGCACCC CCCTCCCCGAGTTGCTGAGCACGGCCCGGCTTCGGGTGC GGGGCTCCGTACGGGGCGTGGCGCGGGGCTCGCCGTGC CGGGCGGGGGGTGGCGGCAGGTGGGGGTGCCGGGCGG GGCGGGGCCGCCTCGGGCCGGGGAGGGCTCGGGGGAG GGGCGCGGCGGCCCCCGGAGCGCCGGCGGCTGTCGAGG CGCGGCGAGCCGCAGCCATTGCCTTTTATGGTAATCGTG CGAGAGGGCGCAGGGACTTCCTTTGTCCCAAATCTGTGC GGAGCCGAAATCTGGGAGGCGCCGCCGCACCCCCTCTA GCGGGCGCGGGGCGAAGCGGTGCGGCGCCGGCAGGAA GGAAATGGGCGGGGAGGGCCTTCGTGCGTCGCCGCGCC GCCGTCCCCTTCTCCCTCTCCAGCCTCGGGGCTGTCCGC GGGGGGACGGCTGCCTTCGGGGGGGACGGGGCAGGGC GGGGTTCGGCTTCTGGCGTGTGACCGGCGGCTCTAGAG CCTCTGCTAACCATGTTCATGCCTTCTTCTTTTTCCTACA GCTCCTGGGCAACGTGCTGGTTATTGTGCTGTCTCATCA TTTTGGCAAAGAATTGTGTACAACCATGGAGAGCGACG AGAGCGGCCTGCCCGCCATGGAGATCGAGTGCCGCATC ACCGGCACCCTGAACGGCGTGGAGTTCGAGCTGGTGGG CGGCGGAGAGGGCACCCCCAAGCAGGGCCGCATGACCA ACAAGATGAAGAGCACCAAAGGCGCCCTGACCTTCAGC CCCTACCTGCTGAGCCACGTGATGGGCTACGGCTTCTAC CACTTCGGCACCTACCCCAGCGGCTACGAGAACCCCTTC CTGCACGCCATCAACAACGGCGGCTACACCAACACCCG CATCGAGAAGTACGAGGACGGCGGCGTGCTGCACGTGA GCTTCAGCTACCGCTACGAGGCCGGCCGCGTGATCGGC GACTTCAAGGTGGTGGGCACCGGCTTCCCCGAGGACAG CGTGATCTTCACCGACAAGATCATCCGCAGCAACGCCA CCGTGGAGCACCTGCACCCCATGGGCGATAACGTGCTG GTGGGCAGCTTCGCCCGCACCTTCAGCCTGCGCGACGG CGGCTACTACAGCTTCGTGGTGGACAGCCACATGCACTT CAAGAGCGCCATCCACCCCAGCATCCTGCAGAACGGGG GCCCCATGTTCGCCTTCCGCCGCGTGGAGGAGCTGCACA GCAACACCGAGCTGGGCATCGTGGAGTACCAGCACGCC TTCAAGACCCCCATCGCCTTCGCCAGATCCCGCGCTCAG TCGTCCAATTCTGCCGTGGACGGCACCGCCGGACCCGG CTCCACCGGATCTCGCTAAACGCGTGAATTCACTCCTCA GGTGCAGGCTGCCTATCAGAAGGTGGTGGCTGGTGTGG CCAATGCCCTGGCTCACAAATACCACTGAGATCTTTTTC CCTCTGCCAAAAATTATGGGGACATCATGAAGCCCCTTG AGCATCTGACTTCTGGCTAATAAAGGAAATTTATTTTCA TTGCAATAGTGTGTTGGAATTTTTTGTGTCTCTCACTCGG AAGGACATATGGGAGGGCAAATCATTTAAAACATCAGA ATGAGTATTTGGTTTAGAGTTTGGCAACATATGCCCATA TGCTGGCTGCCATGAACAAAGGTTGGCTATAAAGAGGT CATCAGTATATGAAACAGCCCCCTGCTGTCCATTCCTTA TTCCATAGAAAAGCCTTGACTTGAGGTTAGATTTTTTTT ATATTTTGTTTTGTGTTATTTTTTTCTTTAACATCCCTAA |
| | | AATTTTCCTTACATGTTTTACTAGCCAGATTTTTCCTCCT CTCCTGACTACTCCCAGTCATAGCTGTCCCTCTTCTCTTA TGGAGATCACCGGTATAACTTCGTATAATGTATACTATA CGAAGTTATGGTACCTTAATTAAGTTAACATGCATAGTA CTGAGCTCACGGGGACAGCCCCCCCCCAAAGCCCCCAG GGATGTAATTACGTCCCTCCCCCGCTAGGGGGCAGCAG CGAGCCGCCCGGGGCTCCGCTCCGGTCCGGCGCTCCCCC CGCATCCCCGAGCCGGCAGCGTGCGGGGACAGCCCGGG CACGGGGAAGGTGGCACGGGATCGCTTTCCTCTGAACG CTTCTCGCTGCTCTTTGAGCCTGCAGACACCTGGGGGGA TACGGGGAAAAAGCTTTAGGCTGAAAGAGAGATTTAGA ATGACAGAATCATAGAACGGCCTGGGTTGCAAAGGAGC ACAGTGCTCATCCAGATCCAACCCCCTGCTATGTGCAGG GTCATCAACCAGCAGCCCAGGCTGCCCAGAGCCACATC CAGCCTGGCCTTGAATGCCTGCAGGGATGGGGCATCCA CAGCCTCCTTGGGCAACCTGTTCAGTGCGTCACCACCCT CTGGGGGAAAAACTGCCTCCTCATATCCAACCCAAACC TCCCCTGTCTCAGTGTAAAGCCATTCCCCCTTGTCCTATC AAGGGGGAGTTTGCTGTGACATTGTTGGTCTGGGGTGA CACATGTTTGCCAATTCAGTGCATCACGGAGAGGCAGA TCTTGGGGATAAGGAAGTGCAGGACAGCATGGACGTGG GACATGCAGGTGTTGAGGGCTCTGGGACACTCTCCAAG TCACAGCGTTCAGAACAGCCTTAAGGATAAGAAGATAG GATAGAAGGACAAAGAGCAAGTTAAAACCCAGCATGG AGAGGAGCACAAAAAGGCCACAGACACTGCTGGTCCCT GTGTCTGAGCCTGCATGTTTGATGGTGTCTGGATGCAAG CAGAAGGGGTGGAAGAGCTTGCCTGGAGAGATACAGCT GGGTCAGTAGGACTGGGACAGGCAGCTGGAGAATTGCC ATGTAGATGTTCATACAATCGTCAAATCATGAAGGCTG GAAAAGCCCTCCAAGATCCCCAAGACCAACCCCAACCC ACCCACCGTGCCCACTGGCCATGTCCCTCAGTGCCACAT CCCCACAGTTCTTCATCACCTCCAGGGACGGTGACCCCC CCACCTCCGTGGGCAGCTGTGCCACTGCAGCACCGCTCT TTGGAGAAGGTAAATCTTGCTAAATCCAGCCCGACCCTC CCCTGGCACAACGTAAGGCCATTATCTCTCATCCAACTC CAGGACGGAGTCAGTGAGAATATTGTTTAAACACTAGG GACAGGATTGGTGACAGAAAAGCCCCATCCTTAGGCCT CCTCCTTCCTAGTCTCCTGATATTGGGTCTAACCCCCAC CTCCTGTTAGGCAGATTCCTTATCTGGTGACACACCCCC ATTTCCTGGAGCCATCTCTCTCCTTGCCAGAACCTCTAA GGTTTGCTTACGATGGAGCCAGAGAGGATCCTGGGAGG GAGAGCTTGGCAGGGGGTGGGAGGGAAGGGGGGGATG CGTGACCTGCCCGGTTCTCAGTGGCCACCCTGCGCTACC CTCTCCCAGAACCTGAGCTGCTCTGACGCGGCTGTCTGG TGCGTTTCACTGATCCTGGTGCTGCAGCTTCCTTACACTT CCCAAGAGGAGAAGCAGTTTGGAAAAACAAAATCAGA ATAAGTTGGTCCTGAGTTCTAACTTTGGCTCTTCACCTTT CTAGTCCCCAATTTATATTGTTCCTCCGTGCGTCAGTTTT ACCTGTGAGATAAGGCCAGTAGCCAGCCCCGTCCTGGC AGGGCTGTGGTGAGGAGGGGGGTGTCCGTGTGGAAAAC TCCCTTTGTGAGAATGGTGCGTCCTAGGTGTTCACCAGG TCGTGGCCGCCTCTACTCCCTTTCTCTTTCTCCATCCTTC TTTCCTTAAAGAGTCCCCAGTGCTATCTGGGACATATTC CTCCGCCCAGAGCAGGGTCCCGCTTCCCTAAGGCCCTGC |
| | | TCTGGGCTTCTGGGTTTGAGTCCTTGGCAAGCCCAGGAG AGGCGCTCAGGCTTCCCTGTCCCCCTTCCTCGTCCACCA TCTCATGCCCCTGGCTCTCCTGCCCCTTCCCTACAGGGG TTCCTGGCTCTGCTCTGTTTAAACACTGGCCGTCGTTTTA CAACGTCGTGACTGGGAAAACCCTGGCGTTACCCAACT TAATCGCCTTGCAGCACATCCCCCTTTCGCCAGCTGGCG TAATAGCGAAGAGGCCCGCACCGATCGCCCTTCCCAAC AGTTGCGCAGCCTGAATGGCGAATGGCGCCTGATGCGG TATTTTCTCCTTACGCATCTGTGCGGTATTTCACACCGCA TATGGTGCACTCTCAGTACAATCTGCTCTGATGCCGCAT AGTTAAGCCAGCCCCGACACCCGCCAACACCCGCTGAC GCGCCCTGACGGGCTTGTCTGCTCCCGGCATCCGCTTAC AGACAAGCTGTGACCGTCTCCGGGAGCTGCATGTGTCA GAGGTTTTCACCGTCATCACCGAAACGCGCGA |

### [Example 5]

### Introduction of GFP expression cassette of donor vector into AAVS1, GSH1, GSH2, and GSH3 sites in genome and expression verification

293T cells were used to validate the introduction and expression of the GFP gene. The 293T cells were cultured in Dulbecco's modified Eagle's medium (Gibco) supplemented with 10% fetal bovine serum (v/v), penicillin (100 U/ml), and streptomycin (100 µg/ml).

293T cells were seeded at a density of 5 x 10⁴ /well on a 24-well culture dish one day before transfection and incubated in a CO₂ incubator for 24 hours. After 24 hours, the medium was replaced with a 293T cell culture medium that does not contain antibiotics. 25 µl of Opti-MEM (Gibco) was seeded into two 1.5 ml tubes. 650 ng of a gRNA/Cas9 vector was mixed with 1,300 ng of a donor vector in one tube (Tube 1). 2 µl of Lipofectamine 2000 was mixed in the other tube (Tube 2). The solution in Tube 2 was transferred to Tube 1, followed by mixing and incubating at room temperature for 10 minutes. After 10 minutes, approximately 50 µl of the solution containing the vector was added to the 293T cells in a 24-well culture dish to induce transfection. After 48 hours, the medium was replaced with fresh culture medium.

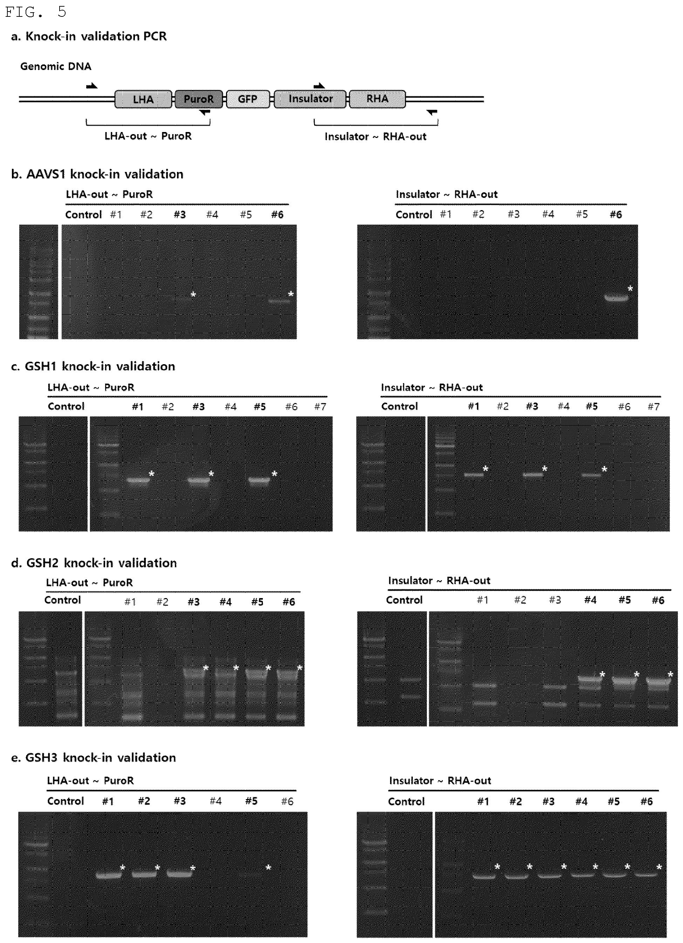

The transfected cells were seeded at a density of 1,000 cells/100 mm culture dish and single-cell-derived colonies expressing GFP were selected after 2 weeks. 6, 7, 6, and 6 colonies were selected from 293T cells that introduced GFP expression cassettes into AAVS1, GSH1, GSH2, and GSH3, respectively, PCR was performed as shown in FIG. 5A to validate the introduction of transgenes due to HDR, and the results are shown in FIGs. 5B to 5E.

In addition, as shown in Table 5 below, clones in which gene introduction was validated were 16.67%, 42.85%, 50%, and 66.67% in AAVS1, GSH1, GSH2, and GSH3, respectively.

**[Table 5]**

| GSH | Knock-in % |
|---|---|
| AAVS1 | 16.67 |
| GSH1 | 42.85 |
| GSH2 | 50.00 |
| GSH3 | 66.67 |

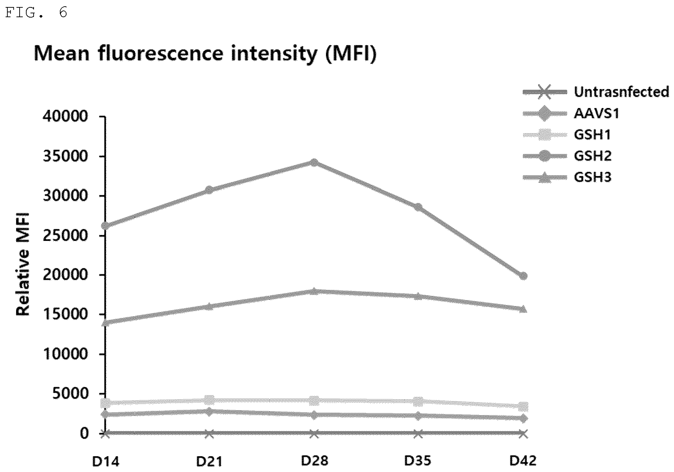

To validate the expression stability of the introduced GFP gene, the mean fluorescence intensity (MFI) of GFP was measured once a week for 4 weeks using flow cytometry. As shown in FIG. 6, when GFP was introduced into GSH1, GSH2, and GSH3, MFI was high, compared to when GFP was introduced into AAVS1.

### [Example 6]

### Determination of changes in expression of adjacent genes

It was validated whether or not stable expression of transgenes could be induced without transcriptional disturbance of the adjacent genes when the transgenes were introduced into the GSH position. The expression changes of 10 adjacent genes in AAVS1, 3 adjacent genes in GSH1, 1 adjacent gene in GSH2, and 4 adjacent genes in GSH3 were analyzed using real-time PCR. The AAVS1, GSH1, GSH2, and GSH3 positions, and distances between adjacent genes are shown in Table 6.

Total RNA was extracted from 293T cells that had GFP expression cassettes introduced into AAVS1, GSH1, GSH2, and GSH3 using a PureLink RNA mini kit (Invitrogen). The extracted RNA was assayed and complementary DNA (cDNA) was synthesized using AccuPower RT Master Mix (BIONEER). 100 ng of cDNA was analyzed using TB Green Premix Ex Taq ± in QuantStudio 1 real-time PCR system (Thermo Fisher) to measure the relative expression of each gene. Genes (IZUMO3) having Ct of 37 or more were excluded.

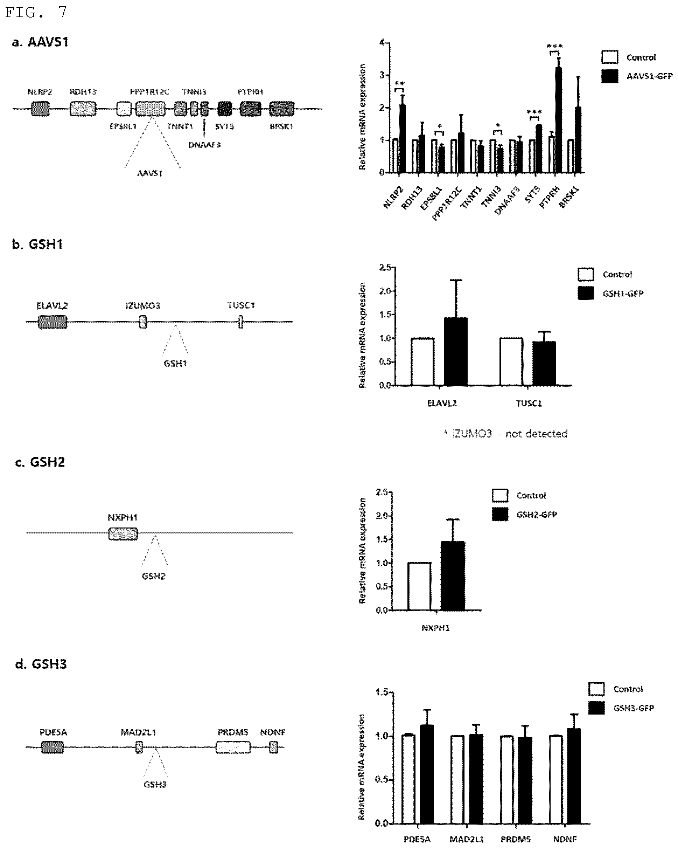

As shown in FIG. 7A, the expression of 5 out of 10 genes adjacent to AAVS1 (NLRP2, EPS8L1, TNNI3, SYT5, and PTPRH) significantly increased or decreased. As shown in FIG. 7B, the expression of genes adjacent to GSH1 did not have any significant difference. As shown in FIG. 7C, the expression of the gene NXPH1 adjacent to GSH2 did not have a significant difference. As shown in FIG. 7D, the expression of all four genes adjacent to GSH3 did not have a significant difference.

Therefore, it can be seen that the GSH1, GSH2, and GSH3 positions have higher gene expression efficiency than AAVS1 and do not affect the transcription of adjacent genes and thus have excellent safety when transgenes are introduced.

**[Table 6]**

| Candidate | Chromosome | Gene | Start | End | Distance from GSH |
|---|---|---|---|---|---|
| AAVS1 | 19 | NLRP2 | 54966374 | 55001138 | 114633 |
| | | RDH13 | 55039108 | 55071291 | 44480 |
| | | EPS8L1 | 55072020 | 55087923 | 27848 |
| | | PPP1R12C | 55090914 | 55117637 | - |
| | | TNNT1 | 55132698 | 55149206 | 16909 |
| | | TNNI3 | 55151767 | 55157773 | 35978 |
| | | DNAAF3 | 55158661 | 55166722 | 42872 |
| | | SYT5 | 55171196 | 55180289 | 55407 |
| | | PTPRH | 55181247 | 55209506 | 65458 |
| | | BRSK1 | 5528207 | 5531256 | 16628 |
| GSH1 | 9 | ELAVL2 | 23690104 | 23826337 | 1136326 |
| | | IZUMO3 | 24542952 | 24545946 | 416717 |
| | | TUSC1 | 25676969 | 25678444 | 714234 |
| GSH2 | 4 | NXPH1 | 8433609 | 8752961 | 311315 |
| GSH3 | 3 | PDE5A | 119494397 | 119628804 | 545425 |
| | | MAD2L1 | 120055623 | 120066858 | 107371 |
| | | PRDM5 | 120684919 | 120922870 | 510611 |
| | | NDNF | 121035613 | 121073021 | 861305 |

### [Example 7]

### Evaluation of hair follicle formation after introduction of Sonic hedgehog (SHH) genes into GSH3 site of human embryonic stem cell-derived mesenchymal stem cell (ES-MSC)

### 7.1 Production of SHH expression cassette

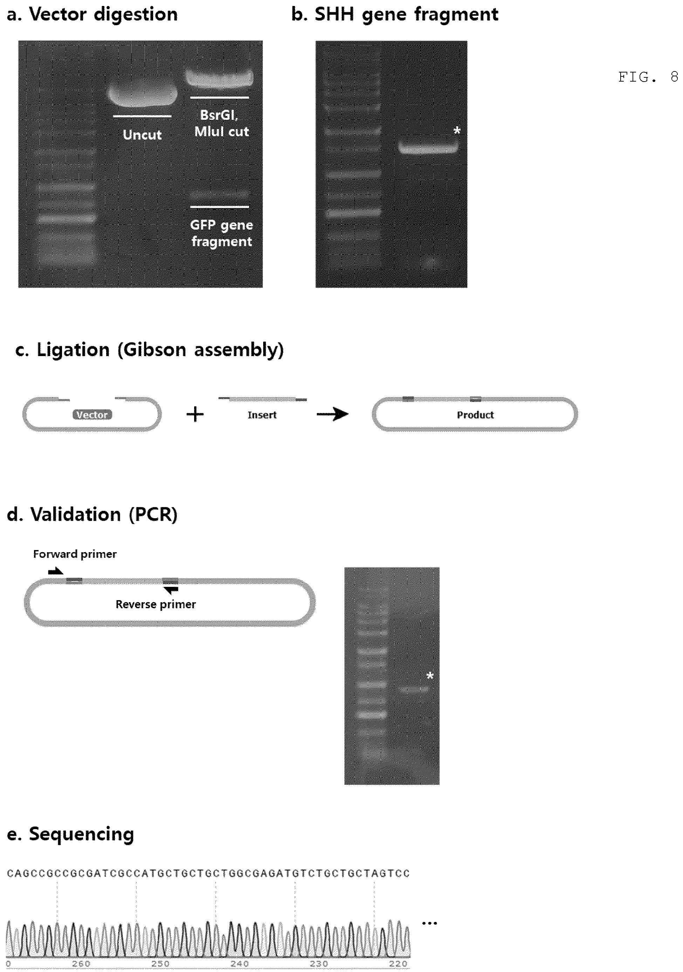

In order to introduce the SHH genes into the GSH3 site of ES-MSC, a donor vector, into which the SHH gene sequence is inserted, instead of GFP, was produced, as shown in FIG. 8. The production of donor vector was performed as follows. First, the GFP gene sequence was removed from the donor vector by restriction enzyme treatment. Then, an SHH gene fragment was produced from an SHH expression vector (OriGene) by PCR. The SHH gene fragment was inserted into the donor vector by Gibson assembly. Whether or not the fragment was introduced into the vector was validated by PCR and a homology DNA sequence introduced into the vector was validated by Sanger sequencing.

### 7.2 Introduction of SHH expression cassette into GSH3

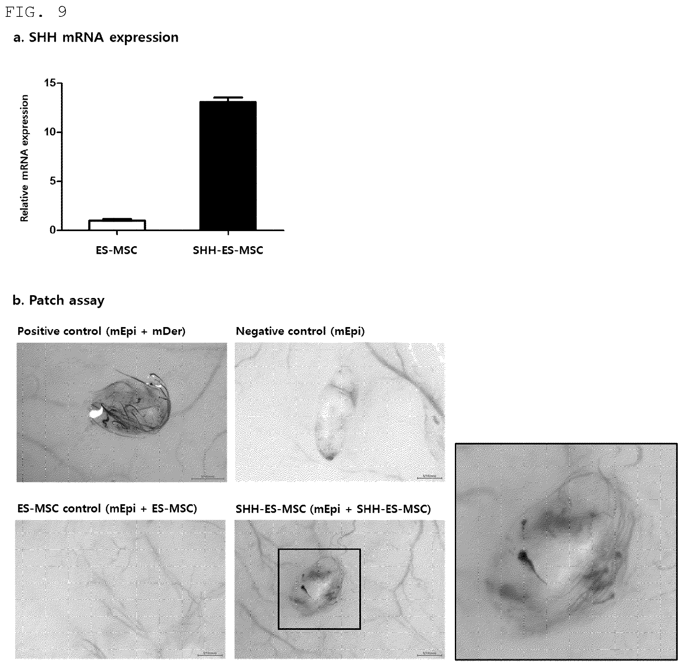

The SHH expression cassette of the donor vector was introduced into the GSH3 site. Then, an experiment was conducted to validate the expression of the SHH expression cassette. ES-MSCs were incubated in StemPro MSC SFM XenoFree medium (Gibco) containing L-glutamine (2 mM), penicillin (100 U/ml), and streptomycin (100 µg/ml). The gRNA/Cas9 expression vector was mixed with the donor vector in a ratio of 1:2 and the resulting mixture was transfected into 2x10⁶ ES-MSC cells using a Neon electroporation device (Invitrogen). The overexpression of the SHH gene was validated by real-time PCR. As shown in FIG. 9A, the result showed that the expression of SHH mRNA in the transfected cells increased about 13-fold.

### 7.3 Evaluation of hair follicle neogenesis of SHH expression cassette

The hair follicle neogenesis of SHH overexpressing ES-MSC (SHH-ES-MSC) was evaluated using patch assay. Epidermal cells and dermal cells were isolated from the skin of newborn C57BL/6 mice. 100 ES-MSC spheroids (n=1 × 10⁴ cells) cultured in an ultra-low attachment 96-well round bottom plate (S-Bio) were mixed with 1 × 10⁶ epidermal cells and the resulting mixture was transplanted subcutaneously on the dorsal side of 6-week-old nude mice (BALB/cAJcl-nu). The group transplanted with the mixture of epidermal and dermal cells was used as a positive control and the group transplanted with only the epidermal cells was used as a negative control. As shown in FIG. 9B, hair follicles were formed in the group transplanted with SHH-ES-MSC 3 weeks after transplantation.

## Claims

1. A method of expressing a exogenous polynucleotide in a cell, the method comprising:
(1) expressing a nuclease by introducing a polynucleotide encoding the nuclease into the cell;
(2) cleaving a nucleic acid region by specifically binding the nuclease to at least one nucleic acid region selected from the group consisting of a first nucleic acid region at positions 24894446 to 24894525 in chromosome 9 of the human genome, a second nucleic acid region at positions 9064276 to 9064355 of chromosome 3, and a third nucleic acid region at positions 120174229 to 120174308 in chromosome 4; and
(3) introducing a exogenous polynucleotide into the cell and inserting the exogenous polynucleotide into a cleavage site of the nucleic acid region.

2. The method according to claim 1, wherein the cell is derived from human blood, bodily fluid, tissue, stem cells, or cancer.

3. The method according to claim 1, wherein the cell is a somatic cell, a germ cell, a stem cell, a cancer cell, or a cell line.

4. The method according to claim 1, wherein the nuclease comprises at least one selected from zinc finger nucleases, transcription activator-like effector nucleases (TALENs), and RNA-guided engineered nucleases (RGENs).

5. The method according to claim 4, wherein the nuclease is Cas9.

6. The method according to claim 1, wherein the polynucleotide encoding the nuclease comprises at least one selected from a DNA binding domain, a guide RNA, and a cleavage domain.

7. The method according to claim 1, further comprising introducing a polynucleotide encoding a guide RNA into the cell.

8. The method according to claim 1, wherein the exogenous polynucleotide comprises a polynucleotide encoding a polypeptide or a polynucleotide encoding a functional polyribonucleotide.

9. The method according to claim 8, wherein the functional polyribonucleotide comprises at least one selected from the group consisting of micro RNA (miRNA), short hairpin RNA (shRNA), piRNA, small nucleolar RNA (snoRNA), small nuclear RNA (snRNA), and extracellular RNA (exRNA).

10. The method according to claim 8, wherein the exogenous polynucleotide encodes at least one selected from the group consisting of antibodies, enzymes, growth factors, receptors, hormones, lymphokines, cytokines, signaling factors, reporters, and fragments thereof.

11. The method according to claim 8, wherein the exogenous polynucleotide is a sonic hedgehog (SHH) gene.

12. The method according to claim 8, wherein the exogenous polynucleotide comprises at least one selected from the group consisting of an open reading frame, a polyadenylation sequence, a promoter, an operator, an enhancer, a transcriptional regulatory element, a signal sequence, and at least one homology region.

13. The method according to claim 1, further comprising introducing a left homology arm (LHA), which is a region binding to a region to up to 1 kb away from the left of at least one of cleavage sites of the first to third nucleic acid regions, and a right homology arm (RHA), which is a region binding to a region to up to 1 kb away from the right of at least one of the cleavage sites of the first to third nucleic acid regions, into the exogenous polynucleotide.

14. The method according to claim 13, further comprising introducing a left homology arm (LHA), which is a region binding to a region to up to 0.8 kb away from the left of at least one of cleavage sites of the first to third nucleic acid regions, and a right homology arm (RHA), which is a region binding to a region to up to 0.8 kb away from the right of at least one of the cleavage sites of the first to third nucleic acid regions, into the exogenous polynucleotide.

15. A cell having a exogenous polynucleotide inserted into a genome of a cell by at least one nuclease,
wherein the exogenous polynucleotide is inserted into at least one selected from a first nucleic acid region from positions 24894446 to 24894525 in chromosome 9 of the human genome, a second nucleic acid region from positions 9064276 to the 9064355 in chromosome 3, and a third nucleic acid region from positions 120174229 to 120174308 in chromosome 4 in the genome of the cell.

16. A composition for expressing a exogenous polynucleotide in a cell, the composition comprising:
a polynucleotide encoding a nuclease that specifically binds to at least one selected from a first nucleic acid region from positions 24894446 to 24894525 in chromosome 9 of the human genome, a second nucleic acid region from positions 9064276 to 9064355 in chromosome 3, and a third nucleic acid region from positions 120174229 to 120174308 in chromosome 4; and
a exogenous polynucleotide.

17. The composition according to claim 16, further comprising:
a left homology arm (LHA), which is a region binding to a region to up to 1 kb away from the left of at least one of cleavage sites of the first to third nucleic acid regions; and
a right homology arm (RHA), which is a region binding to a region to up to 1 kb away from the right of at least one of the cleavage sites of the first to third nucleic acid regions.

18. The composition according to claim 17, further comprising:
a left homology arm (LHA), which is a region binding to a region to up to 0.8 kb away from the left of at least one of cleavage sites of the first to third nucleic acid regions; and
a right homology arm (RHA), which is a region binding to a region to up to 0.8 kb away from the right of at least one of the cleavage sites of the first to third nucleic acid regions.

19. The composition according to claim 16, wherein the exogenous polynucleotide is a sonic hedgehog (SHH) gene.

20. A polynucleotide comprising a sequence having a sequence homology of 90% or more with any one of sequences of SEQ ID NOs: 3 to 5.

21. The polynucleotide according to claim 20, wherein the polynucleotide comprises a sequence having a sequence homology of 95% or more with any one of sequences of SEQ ID NOs: 3 to 5.

22. The polynucleotide according to claim 20, wherein the polynucleotide comprises a sequence having a sequence homology of 99% or more with any one of sequences of SEQ ID NOs: 3 to 5.

23. The polynucleotide according to claim 20, wherein the polynucleotide comprises any one of sequences of SEQ ID NOs: 3 to 5.

24. The polynucleotide according to claim 20, wherein the sequence of SEQ ID NO: 3 specifically binds to a nucleic acid region at positions 24894446 to 24894525 in chromosome 9 of the human genome.

25. The polynucleotide according to claim 20, wherein the sequence of SEQ ID NO: 4 specifically binds to a nucleic acid region at positions 9064276 to 9064355 in chromosome 3 of the human genome.

26. The polynucleotide according to claim 20, wherein the sequence of SEQ ID NO: 5 specifically binds to a nucleic acid region at positions 120174229 to 120174308 in chromosome 4 of the human genome.

27. A polynucleotide comprising sequences of SEQ ID NOs: 14 and 15.

28. A polynucleotide comprising sequences of SEQ ID NOs: 16 and 17.

29. A polynucleotide comprising sequences of SEQ ID NOs: 18 and 19.

30. A vector comprising the polynucleotide according to any one of claims 27 to 29.
